# EUROPÄISCHE PATENTSCHRIFT

(11) **EP 1 731 511 B1**
(45) Veröffentlichungstag und Bekanntmachung des Hinweises auf die Patenterteilung: **12.08.2015**
(21) Anmeldenummer: 06116986.8
(22) Anmeldetag: 16.06.2000
(51) Int. Cl.: C07D 239/94, C07D 215/54, C07D 405/12, A61K 31/517, A61P 35/00

(54) **Bicyclische Heterocyclen, diese Verbindungen enthaltende Arzneimittel, deren Verwendung und Verfahren zu ihrer Herstellung**
Bicyclic heterocycles, medicaments containing these compounds, their use and methods for the production thereof
Heterocycles bicycliques, medicaments contenant ces composés, leur utilisation et procédés permettant de les préparer

(30) Priorität: 21.06.1999 DE 19928281; 30.07.1999 US 146644 P; 11.05.2000 DE 10023085
(43) Veröffentlichungstag der Anmeldung: 13.12.2006
(62) Teilanmeldung aus: 00936888.7
(73) Patentinhaber: Boehringer Ingelheim Pharma GmbH & Co. KG, 55216 Ingelheim (DE)
(72) Erfinder: Himmelsbach, Frank, 88441 Mittelbiberach (DE); Langkopf, Elke, 88447 Warthausen (DE); Jung, Birgit, 88471 Laupheim (DE); Baum, Anke, 1050 Wien (AT); Solca, Flavio, 1230 Wien (AT); Metz, Thomas, 79286 Glottertal (DE)

(56) Entgegenhaltungen:
- WO-A-97/38983
- WO-A-98/43960
- WO-A-99/06378

## Beschreibung

Gegenstand der vorliegenden Erfindung sind bicyclische Heterocyclen der allgemeinen Formel 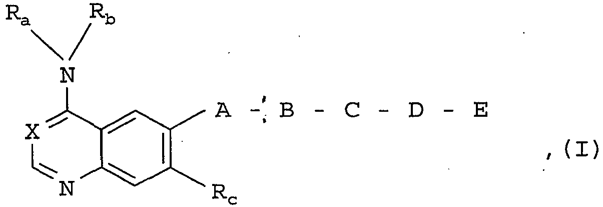 deren Tautomeren, deren Stereoisomere und deren Salze, insbesonders deren physiologisch verträgliche Salze mit anorganischen oder organischen Säuren oder Basen, welche wertvolle pharmakologische Eigenschaften aufweisen, insbesondere eine Hemmwirkung auf die durch Tyrosinkinasen vermittelte Signaltransduktion, deren Verwendung zur Behandlung von Krankheiten, insbesondere von Tumorerkrankungen, von Erkrankungen der Lunge und der Atemwege und deren Herstellung.

In der obigen allgemeinen Formel I bedeutet
Rₐ ein Wasserstoffatom,
R_{b} eine 1-Phenylethylgruppe oder eine Phenylgruppe, in der der Phenylkern durch die Reste R₁ und R₂ substituiert ist, wobei
R₁ und R₂, die gleich oder verschieden sein können, jeweils ein Wasserstoff-, Fluor-, Chlor- oder Bromatom darstellen,
X ein Stickstoffatom,
A eine Iminogruppe,
B eine Carbonylgruppe,
C eine 1,2-Vinylen- oder Ethinylengruppe,
D eine Methylengruppe,
E eine Dimethylamino-, Diethylamino-, Bis-(2-methoxy-ethyl)-amino-, N-Methyl-N-(2-methoxy-ethyl)-amino-, N-Ethyl-N-(2-methoxy-ethyl)-amino-, N-Methyl-N-cyclopropyl-amino-, N-Methyl-N-cyclopropylmethyl-amino-, N-Methyl-N-(1-methoxy-2-propyl)-amino-, N'-Methyl-N-(2-methoxy-propyl)-amino-, N-Me-thyl-N-(3-methoxy-propyl)-amino-gruppe,
eine gegebenenfalls durch eine oder zwei Methylgruppen substituierte Pyrrolidino-, Piperidino- oder.Morpholinogruppe, und
R_{c} eine Cyclopropylmethoxy-, Cyclobutyloxy- oder Cyclopentyloxygruppe,
eine Tetrahydrofuran-3-yloxy-, Tetrahydropyran-4-yloxy- oder Tetrahydrofuran-2-ylmethoxygruppe bedeuten,
deren Tautomere, deren Stereoisomere und deren Salze.

Beispielsweise seien folgende besonders bevorzugte Verbindungen der allgemeinen Formel I erwähnt:
(1) 4-[(3-Chlor-4-fluorphenyl)amino]-6-{[4-(N,N-diethylamino)-1-oxo-2-buten-1-yl]amino}-7-cyclopropylmethoxy-chinazolin,
(2) 4-[(3-Chlor-4-fluorphenyl)amino]-6-{[4-(morpholin-4-yl)-1-oxo-2-buten-1-yl]amino}-7-cyclopropylmethoxy-chinazolin,
(3) 4-[(3-Chlor-4-fluorphenyl)amino]-6-{[4-(2,6-dimethyl-morpholin-4-yl)-1-oxo-2-buten-1-yl]amino}-7-cyclopropylmethoxychinazolin,
(4) 4-[(3-Chlor-4-fluorphenyl)amino]-6-{[4-(dimethylamino)-1-oxo-2-buten-1-yl]amino}-7-cyclopropylmethoxy-chinazolin,
(5) 4-[(3-Chlor-4-fluorphenyl)amino]-6-{[4-(morpholin-4-yl)-1-oxo-2-buten-1-yl]amino}-7-cyclobutyloxy-chinazolin,
(6) 4-[(3-Chlor-4-fluorphenyl)amino]-6-{[4-(morpholin-4-yl)-1-oxo-2-buten-1-yl]amino}-7-cyclopentyloxy-chinazolin,
(7) 4-[(3-Chlor-4-fluorphenyl)amino]-6-{[4-(diethylamino)-1-oxo-2-buten-1-yl]amino}-7-cyclobutyloxy-chinazolin,
(8) 4-[(3-Chlor-4-fluorphenyl)amino]-6-{[4-(diethylamino)-1-oxo-2-buten-1-yl]amino}-7-cyclopentyloxy-chinazolin,
(9) 4-[(R)-(1-Phenyl-ethyl)amino]-6-{[4-(morpholin-4-yl)-1-oxo-2-buten-1-yl]amino}-7-cyclobutyloxy-chinazolin,
(10) 4-[(R)-(1-Phenyl-ethyl)amino]-6-{[4-(morpholin-4-yl)-1-oxo-2-buten-1-yl]amino}-7-cyclopropylmethoxy-chinazolin,
(11) 4-[(R)-(1-Phenyl-ethyl)amino]-6-{[4-(morpholin-4-yl)-1-oxo-2-buten-1-yl]amino}-7-cyclopentyloxy-chinazolin,
(12) 4-[(R)-(1-Phenyl-ethyl)amino]-6-{[4-(diethylamino)-1-oxo-2-buten-1-yl]amino}-7-cyclobutyloxy-chinazolin,
(13) 4-[(R)-(1-Phenyl-ethyl)amino]-6-{[4-(diethylamino)-1-oxo-2-buten-1-yl]amino}-7-cyclopentyloxy-chinazolin,
(14) 4-[(R)-(1-Phenyl-ethyl)amino]-6-{[4-(diethylamino)-1-oxo-2-buten-1-yl]amino}-7-cyclopropylmethoxy-chinazolin,
(15) 4-[(3-Chlor-4-fluorphenyl)amino]-6-({4-[bis-(2-methoxy-ethyl)-amino]-1-oxo-2-buten-1-yl}amino)-7-cyclopropylmethoxy-chinazolin,
(16) 4-[(3-Chlor-4-fluorphenyl)amino]-6-({4-[N-ethyl-N-(2-methoxyethyl)-amino]-1-oxo-2-buten-1-yl}amino)-7-cyclopropyl-methoxy-chinazolin,
(17) 4-[(3-Chlor-4-fluorphenyl)amino]-6-{[4-(piperidin-1-yl)-1-oxo-2-buten-1-yl]amino}-7-cyclopropylmethoxy-chinazolin,
(18) 4-[(3-Chlor-4-fluorphenyl)amino]-6-{[4-(2-methyl-piperidin-1-yl)-1-oxo-2-buten-1-yl]amino}-7-cyclopropylmethoxychinazolin,
(19) 4-[(3-Chlor-4-fluorphenyl)amino]-6-{[4-(pyrrolidin-1-yl)-1-oxo-2-buten-1-yl]amino}-7-cyclopropylmethoxy-chinazolin,
(20) 4-[(3-Chlor-4-fluorphenyl)amino]-6-{[4-(2-methyl-pyrrolidin-1-yl)-1-oxo-2-buten-1-yl]amino}-7-cyclopropylmethoxy-chinazolin,
(21) 4-[(3-Chlor-4-fluorphenyl)amino]-6-{[4-(cis-2,6-dimethyl-piperidin-1-yl)-1-oxo-2-buten-1-yl]amino}-7-cyclopropylmethoxy-chinazolin,
(22) 4-[(3-Chlor-4-fluorphenyl)amino]-6-{[4-(2,5-dimethyl-pyrrolidin-1-yl)-1-oxo-2-buten-1-yl]amino}-7-cyclopropylmethoxy-chinazolin,
(23) 4-[(3-Chlor-4-fluorphenyl)amino]-6-{[4-(diethylamino)-1-oxo-2-buten-1-yl]amino}-7-[(tetrahydrofuran-2-yl)methoxy]-chinazolin,
(24) 4-[(3-Chlor-4-fluorphenyl)amino]-6-{[4-(diethylamino)-1-oxo-2-buten-1-yl]amino}-7-[(S)-(tetrahydrofuran-3-yl)oxy]-chinazolin,
(25) 4-[(3-Chlor-4-fluorphenyl)amino]-6-{[4-(4-diethylamino-methyl-piperidin-1-yl)-1-oxo-2-buten-1-yl]amino]-7-cyclopropylmethoxy-chinazolin,
(26) 4-[(3-Chlor-4-fluorphenyl)amino]-6-{[4-(N-methyl-N-cyclopropylmethyl-amino)-1-oxo-2-buten-1-yl]amino}-7-cyclopropylmethoxy-chinazolin,
(27) 4-[(3-Chlor-4-fluorphenyl)amino]-6-({4-[N-methyl-N-(2-methoxypropyl)-amino]-1-oxo-2-buten-1-yl]amino)-7-cyclopropylmethoxy-chinazolin,
(28) 4-[(3-Chlor-4-fluorphenyl)amino]-6-({4-[N-methyl-N-(3-methoxypropyl)-amino]-1-oxo-2-buten-1-yl}amino)-7-cyclopropylmethoxy-chinazolin,
(29) 4-[(3-Chlor-4-fluorphenyl)amino]-6-{[4-(diethylamino)-1-oxo-2-buten-1-yl]amino}-7-[(tetrahydropyran-4-yl)oxy]-chinazolin,
(30) 4-[(3-Chlor-4-fluorphenyl)amino]-6-({4-[N-methyl-N-(1-methoxy-2-propyl)-amino]-1-oxo-2-buten-1-yl}amino)-7-cyclopropylmethoxy-chinazolin,
(31) 4-[(3-Chlor-4-fluorphenyl)amino]-6-{[4-(N,N-dimethylamino)-1-oxo-2-butin-1-yl]amino}-7-cyclopropylmethoxy-chinazolin,
(32) 4-[(3-Chlor-4-fluorphenyl)amino]-6-{[4-(N,N-diethylamino)-1-oxo-2-butin-1-yl]amino}-7-cyclopropylmethoxy-chinazolin,
(33) 4-[(3-Chlor-4-fluorphenyl)amino]-6-{[4-(piperidin-1-yl)-1-oxo-2-butin-1-yl]amino}-7-cyclopropylmethoxy-chinazolin,
(34) 4-[(3-Chlor-4-fluorphenyl)amino]-6-{[4-(morpholin-4-yl)-1-oxo-2-butin-1-yl]amino}-7-cyclopropylmethoxy-chinazolin,
(35) 4-[(3-Chlor-4-fluorphenyl)amino]-6-{[4-(N,N-dimethylamino)-1-oxo-2-buten-1-yl]aminol-7-(2-cyclopropyl-ethoxy)-chinazolin,
(36) 4-[(3-Chlor-4-fluorphenyl)amino]-6-({4-[bis-(2-methoxyethyl)-amino]-1-oxo-2-buten-1-yl}amino)-7-cyclobutyloxy-chinazolin,
(37) 4-[(3-Chlor-4-fluorphenyl)amino]-6-({4-[N-methyl-N-(2-methoxyethyl)-amino]-1-oxo-2-buten-1-yl}amino)-7-cyclobutyloxychinazolin,
(38) 4-[(3-Chlor-4-fluorphenyl)amino]-6-({4-[(S)-N-methyl-N-(1-methoxy-2-propyl)-amino]-1-oxo-2-buten-1-yl}amino)-7-cyclobutyloxy-chinazolin,
(39) 4-[(3-Chlor-4-fluorphenyl)amino]-6-({4-[(R)-N-methyl-N-(1-methoxy-2-propyl)-amino]-1-oxo-2-buten-1-yl}amino)-7-cyclobutyloxy-chinazolin,
(40) 4-[(3-Chlor-4-fluorphenyl)amino]-6-({4-[N-methyl-N-(1-methoxy-2-propyl)-amino]-1-oxo-2-buten-1-yl}amino)-7-cyclopropylmethoxy-chinazolin,
(41) 4-[(3-Chlor-4-fluorphenyl)amino]-6-({4-[N-methyl-N-(2-methoxypropyl)-amino]-1-oxo-2-buten-1-yl}amino)-7-cyclopropylmethoxy-chinazolin,
(42) 4-[(3-Chlor-4-fluorphenyl)amino]-6-({4-[N-methyl-N-(3-methoxypropyl) -amino]-1-oxo-2-buten-1-yl}amino)-7-cyclopropylmethoxy-chinazolin,
(43) 4-[(3-Chlor-4-fluorphenyl)amino]-6-{[4-(N-cyclopropyl-N-methyl-amino)-1-oxo-2-buten-1-yl]amino}-7-cyclobutyloxy-chinazolin,
(44) 4-[(3-Chlor-4-fluorphenyl)amino]-6-{[4-(N-cyclopropylmethyl-N-methyl-amino)-1-oxo-2-buten-1-yl]amino}-7-cyclobutyloxy-chinazolin,
(45) 4-[(3-Chlor-4-fluorphenyl)amino]-6-{[4-(pyrrolidin-1-yl)-1-oxo-2-buten-1-yl]amino}-7-cyclobutyloxy-chinazolin,
(46) 4-[(3-Chlor-4-fluorphenyl)amino]-6-{[4-(2-methyl-pyrrolidin-1-yl)-1-oxo-2-buten-1-yl]amino}-7-cyclobutyloxy-chinazolin,
(47) 4-[(3-Chlor-4-fluorphenyl)amino]-6-{[4-(2,5-dimethyl-pyrrolidin-1-yl)-1-oxo-2-buten-1-yl]amino}-7-cyclobutyloxychinazolin,
(48) 4-[(3-Chlor-4-fluorphenyl)amino]-6-{[4-(piperidin-1-yl)-1-oxo-2-buten-1-yl]amino}-7-cyclobutyloxy-chinazolin,
(49) 4-[(3-Chlor-4-fluorphenyl)amino]-6-{[4-(2-methyl-piperidin-1-yl)-1-oxo-2-buten-1-yl]amino}-7-cyclobutyloxychinazolin,
(50) 4-[(3-Chlor-4-fluorphenyl)amino]-6-{[4-(2,6-dimethyl-piperidin-1-yl)-1-oxo-2-buten-1-yl]amino}-7-cyclobutyloxychinazolin,
(51) 4-[(3-Chlor-4-fluorphenyl)amino]-6-{[4-(3-methyl-morpholin-4-yl)-1-oxo-2-buten-1-yl]amino}-7-cyclobutyloxychinazolin,
(52) 4-[(3-Chlor-4-fluorphenyl)amino]-6-{[4-(3,5-dimethyl-morpholin-4-yl)-1-oxo-2-buten-1-yl]amino}-7-cyclobutyloxy-chinazolin,
(53) 4-[(3-Chlor-4-fluorphenyl)amino]-6-({4-[N-methyl-N-(2-methoxyethyl)-amino]-1-oxo-2-buten-1-yl}amino)-7-(tetrahydrofuran-3-yl-oxy)-chinazolin,
(54) 4-[(3-Chlor-4-fluorphenyl)amino]-6-({4-[N-methyl-N-(2-methoxyethyl)-amino]-1-oxo-2-buten-1-yl}amino)-7-(tetrahydropyran-4-yl-oxy)-chinazolin und
(55) 4-[(3-Chlor-4-fluorphenyl)amino]-6-({4-[N-methyl-N-(2-methoxyethyl)-amino]-1-oxo-2-buten-1-yl}amino)-7-(tetrahydrofuran-2-yl-methoxy)-chinazolin,
sowie deren Salze.

Die Verbindungen der allgemeinen Formel I lassen sich beispielsweise nach folgenden Verfahren herstellen:
a) Umsetzung einer Verbindung der allgemeinen Formel 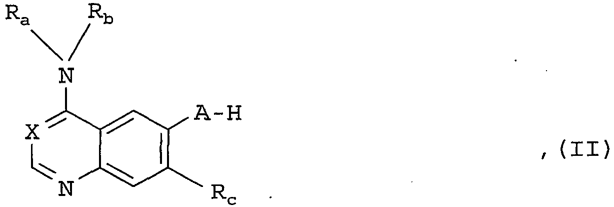 in der
   Rₐ bis R_{c}, A und X wie eingangs erwähnt definiert sind, mit einer Verbindung der allgemeinen Formel

   Z₁ - B - C - D - E, (III)

   in der
   B bis E wie eingangs erwähnt definiert sind und
   Z₁ eine Austrittsgruppe wie ein Halogenatom, z.B. ein Chlor- oder Bromatom, oder eine Hydroxygruppe darstellt.
   Die Umsetzung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan gegebenenfalls in Gegenwart einer anorganischen oder organischen Base und gegebenenfalls in Gegenwart eines wasserentziehenden Mittels zweckmäßigerweise bei Temperaturen zwischen -50 und 150°C, vorzugsweise bei Temperaturen zwischen -20 und 80°C, durchgeführt.
   Mit einer Verbindung der allgemeinen Formel III, in der Z₁ eine Austrittsgruppe darstellt, wird die Umsetzung gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan zeckmäßigerweise in Gegenwart einer tertiären organischen Base wie Triethylamin, Pyridin oder 2-Dimethylaminopyridin, in Gegenwart von N-Ethyl-diisopropylamin (Hünig-Base), wobei diese organischen Basen gleichzeitig auch als Lösungsmittel dienen können, oder in Gegenwart einer anorganischen Base wie Natriumkarbonat, Kaliumcarbonat oder Natronlauge zweckmäßigerweise bei Temperaturen zwischen -50 und 150°C, vorzugsweise bei Temperaturen zwischen -20 und 80°C, durchgeführt.
   Mit einer Verbindung der allgemeinen Formel III, in der Z₁ eine Hydroxygruppe darstellt, wird die Umsetzung vorzugsweise in Gegenwart eines wasserentziehenden Mittels, z.B. in Gegenwart von Chlorameisensäureisobutylester, Thionylchlorid, Trimethylchlorsilan, Phosphortrichlorid, Phosphorpentoxid, Hexamethyldisilazan, N,N'-Dicyclohexylcarbodiimid, N,N'-Dicyclohexylcarbodiimid/N-Hydroxysuccinimid oder 1-Hydroxy-benztriazol und gegebenenfalls zusätzlich in Gegenwart von 4-Dimethylamino-pyridin, N,N'-Carbonyldiimidazol oder Triphenylphosphin/Tetrachlorkohlenstoff zweckmäßigerweise in einem Lösungsmittel wie Methylenchlorid, Tetrahydrofuran, Dioxan, Toluol, Chlorbenzol, Dimethylsulfoxid, Ethylenglycolmonomethylether, Ethylenglycoldiethylether oder Sulfolan und gegebenenfalls in Gegenwart eines Reaktionsbeschleunigers wie 4-Dimethylaminopyridin bei Temperaturen zwischen -50 und 150°C, vorzugsweise jedoch bei Temperaturen zwischen -20 und 80°C, durchgeführt.
b) Zur Herstellung von Verbindungen der allgemeinen Formel I, in der der Rest E über ein Stickstoffatom mit dem Rest D verknüpft ist:
   Umsetzung einer Verbindung der allgemeinen Formel 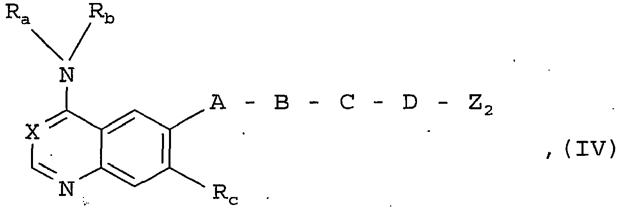 in der
      Rₐ bis R_{c}, A bis D und X wie eingangs erwähnt definiert sind und
      Z₂ eine Austrittsgruppe wie ein Halogenatom, eine substituierte Hydroxy- oder Sulfonyloxygruppe wie ein Chlor- oder Bromatom, eine Methansulfonyloxy- oder p-Toluolsulfonyloxygruppe darstellt, mit einer Verbindung der allgemeinen Formel

      H - E', (V)

      in der
      E' einen der für E eingangs erwähnten Reste darstellt, der über ein Stickstoffatom mit dem Rest D verknüpft ist.

Die Umsetzung wird zweckmäßigerweise in einem Lösungsmittel wie Isopropanol, Butanol, Tetrahydrofuran, Dioxan, Toluol, Chlorbenzol, Dimethylformamid, Dimethylsulfoxid, Methylenchlorid, Ethylenglycolmonomethylether, Ethylenglycoldiethylether oder Sulfolan gegebenenfalls in Gegenwart einer anorganischen oder tertiären organischen Base, z.B. Natriumcarbonat oder Kaliumhydroxid, einer tertiären organischen Base, z.B. Triethylamin, oder in Gegenwart von N-Ethyl-diisopropylamin (Hünig-Base), wobei diese organischen Basen gleichzeitig auch als Lösungsmittel dienen können, und gegebenenfalls in Gegenwart eines Reaktionsbeschleunigers wie einem Alkalihalogenid bei Temperaturen zwischen -20 und 150°C, vorzugsweise jedoch bei Temperaturen zwischen -10 und 100°C, durchgeführt. Die Umsetzung kann jedoch auch ohne Lösungsmittel oder in einem Überschuß der eingesetzten Verbindung der allgemeinen Formel V durchgeführt werden.

Erhält man erfindungsgemäß eine Verbindung der allgemeinen Formel I, die eine Amino-, Alkylamino- oder Iminogruppe enthält, so kann diese mittels Acylierung oder Sulfonylierung in eine entsprechende Acyl- oder Sulfonylverbindung der allgemeinen Formel I übergeführt werden oder

eine Verbindung der allgemeinen Formel I, die eine Amino-, Alkylamino- oder Iminogruppe enthält, so kann diese mittels Alkylierung oder reduktiver Alkylierung in eine entsprechende Alkylverbindung der allgemeinen Formel I übergeführt werden oder
eine Verbindung der allgemeinen Formel I, die eine Carboxy- oder Hydroxyphosphorylgruppe enthält, so kann diese mittels Veresterung in einen entsprechenden Ester der allgemeinen Formel I übergeführt werden oder
eine Verbindung der allgemeinen Formel I, die eine Carboxy- oder Estergruppe enthält, so kann diese durch Umsetzung mit einem Amin in ein entsprechendes Amid der allgemeinen Formel I übergeführt werden.

Die nachträgliche Veresterung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan oder besonders vorteilhaft in einem entsprechenden Alkohol gegebenenfalls in Gegenwart einer Säure wie Salzsäure oder in Gegenwart eines wasserentziehenden Mittels, z.B. in Gegenwart von Chlorameisensäureisobutylester, Thionylchlorid, Trimethylchlorsilan, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid oder 1-Hydroxy-benztriazol und gegebenenfalls zusätzlich in Gegenwart von 4-Dimethylamino-pyridin, N,N'-Carbonyldiimidazol oder Triphenyl-phosphin/Tetrachlorkohlenstoff, zweckmäßigerweise bei Temperaturen zwischen 0 und 150°C, vorzugsweise bei Temperaturen zwischen 0 und 80°C, durchgeführt.

Die nachträgliche Esterbildung kann auch durch Umsetzung einer Verbindung, die eine Carboxy- oder Hydroxyphosphorylgruppe enthält, mit einem entsprechenden Alkylhalogenid erfolgen.

Die nachträgliche Acylierung oder Sulfonylierung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan mit einem entsprechenden Acyl- oder Sulfonylderivat gegebenenfalls in Gegenwart einer tertiärenorganischen Base oder in Gegenwart einer anorganischen Base oder in Gegenwart eines wasserentziehenden Mittels, z.B. in Gegenwart von Chlorameisensäureisobutylester, Thionylchlorid, Trimethylchlorsilan, Schwefelsäure, Methansulfonsäure, p-Toluolsulfonsäure, Phosphortrichlorid, Phosphorpehtoxid, N,N'-Dicyclohexylcarbodiimid, N,N'-Dicyclohexylcarbodiimid/N-Hydroxysuccinimid oder 1-Hydroxy-benztriazol und gegebenenfalls zusätzlich in Gegenwart von 4-Dimethylamino-pyridin, N,N'-Carbonyldiimidazol oder Triphenylphosphin/Tetrachlorkohlenstoff, zweckmäßigerweise bei Temperaturen zwischen 0 und 150°C, vorzugsweise bei Temperaturen zwischen 0 und 80°C, durchgeführt.

Die nachträgliche Alkylierung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan mit einem Alkylierungsmittel wie einem entsprechenden Halogenid oder Sulfonsäureester, z.B. mit Methyljodid, Ethylbromid, Dimethylsulfat oder Benzylchlorid, gegebenenfalls in' Gegenwart einer tertiären organischen Base oder in Gegenwart einer anorganischen Base zweckmäßigerweise bei Temperaturen zwischen 0 und 150°C, vorzugsweise bei Temperaturen zwischen 0 und 100°C, durchgeführt.

Die nachträgliche reduktive Alkylierung wird mit einer entsprechenden Carbonylverbindung wie Formaldehyd, Acetaldehyd, Propionaldehyd, Aceton oder Butyraldehyd in Gegenwart eines komplexen Metallhydrids wie Natriumborhydrid, Lithiumborhydrid, Natriumtriacetoxyborhydrid oder Natriumcyanoborhydrid zweckmäßigerweise bei einem pH-Wert von 6-7 und bei Raumtemperatur oder in Gegenwart eines Hydrierungskatalysators, z.B. mit Wasserstoff in Gegenwart von Palladium/Kohle, bei einem Wasserstoffdruck von 1 bis 5 bar durchgeführt. Die Methylierung kann auch in Gegenwart von Ameisensäure als Reduktionsmittel bei erhöhten Temperaturen, z.B. bei Temperaturen zwischen 60 und 120°C, durchgeführt werden.

Die nachträgliche Amidbildung wird durch Umsetzung eines entsprechenden reaktionsfähigen Carbonsäurederivates mit einem entsprechenden Amin gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan, wobei das eingesetzte Amin gleichzeitig als Lösungsmittel dienen kann, gegebenenfalls in Gegenwart einer tertiären organischen Base oder in Gegenwart einer anorganischen Base oder mit einer entsprechenden Carbonsäure in Gegenwart eines wasserentziehenden Mittels, z.B. in Gegenwart von Chlorameisensäureisobutylester, Thionylchlorid, Trimethylchlorsilan, Phosphortrichlorid, Phosphorpentoxid, N,N'-Dicyclohexylcarbodiimid, N,N'-Dicyclohexylcarbodiimid/N-Hydroxysuccinimid oder 1-Hydroxy-benztriazol und gegebenenfalls zusätzlich in Gegenwart von 4-Dimethylaminopyridin, N,N'-Carbonyldiimidazol oder Triphenylphosphin/Tetrachlorkohlenstoff, zweckmäßigerweise bei Temperaturen zwischen 0 und 150°C, vorzugsweise bei Temperaturen zwischen 0 und 80°C, durchgeführt.

Bei den vorstehend beschriebenen Umsetzungen können gegebenenfalls vorhandene reaktive Gruppen wie Hydroxy-, Carboxy-, Phosphono-, O-Alkyl-phosphono-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Methyl-, Ethyl-, tert-Butyl-, Trityl-, Benzyl- oder Tetrahydropyranylgruppe,
als Schutzreste für eine Carboxygruppe die Trimethylsilyl-, Methyl-, Ethyl-, tert-Butyl-, Benzyl- oder Tetrahydropyranylgruppe,
als Schutzreste für eine Phosphonogruppe eine Alkylgruppe wie die Methyl-, Ethyl-, Isopropyl- oder n-Butylgruppe, die Phenyl- oder Benzylgruppe und
als Schutzreste für eine Amino-, Alkylamino- oder Iminogruppe die Formyl-, Acetyl-, Trifluoracetyl-, Ethoxycarbonyl-, tert.-Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise' jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.-Butyl- oder tert.-Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran bei Temperaturen zwischen 0 und 50°C.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Die Spaltung nur eines Alkylrestes von einer O,O'-Dialkylphosphonogruppe erfolgt beispielsweise mit Natriumiodid in einem Lösungsmittel wie Aceton, Ethyl-methylketon, Acetonitril oder Dimethylformamid bei Temperaburen zwischen 40 und 150°C, vorzugsweise jedoch bei Temperaturen zwischen 60 und 100°C.

Die Abspaltung beider Alkylreste von einer O,O'-Dialkyl-phosphonogruppe erfolgt beispielsweise mit Jodtrimethylsilan, Bromtrimethylsilan oder Chlortrimethylsilan/Natriumiodid in einem Lösungsmittel wie Methylchlorid, Chloroform oder Acetonitril bei Temperaturen zwischen 0°C und der Siedetemperatur des Reaktionsgemisches, vorzugsweise jedoch bei Temperaturen zwischen 20 und 60°C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971)) in ihre optischen Antipoden und Verbindungen der allgemeinen Formel I mit mindestens 2 asymmetrischen Kohlenstoffatomen auf Grund ihrer physikalischchemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxy-, Hydroxyphosphoryl-, Sulfo- oder 5-Tetrazolylgruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Arginin, Cyclohexylamin, Ethanolamin, Diethanolamin und Triethanolamin in Betracht.

Die als Ausgangsstoffe verwendeten Verbindungen der allgemeinen Formeln II bis V sind teilweise literaturbekannt oder man erhält diese nach an sich literaturbekannten Verfahren (siehe Beispiele I bis VI).

Beispielsweise erhält man eine Ausgangsverbindung der allgemeinen Formel II durch Umsetzung einer in 4-Stellung entsprechend substituierten 7-Fluor-6-nitroverbindung mit einem entsprechenden Alkoholat und anschließender Reduktion der so erhaltenen Nitroverbindung oder
eine Ausgangsverbindung der allgemeinen Formel IV durch Umsetzung einer in 4-Stellung entsprechend substituierten 7-Fluor-6-nitroverbindung mit einem entsprechenden Alkoholat, anschließender Reduktion der so erhaltenen Nitroverbindung und anschließend Acylierung mit einer entsprechenden Verbindung.

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf die durch den Epidermal Growth Factor-Rezeptor (EGF-R) vermittelte Signaltransduktion, wobei diese beispielsweise durch eine Inhibition der Ligandenbindung, der Rezeptordimerisierung oder der Tyrosinkinase selbst bewirkt werden kann. Außerdem ist es möglich, daß die Signalübertragung an weiter abwärtsliegenden Komponenten blockiert wird.

Die biologischen Eigenschaften der neuen Verbindungen wurden wie folgt geprüft:
Die Hemmung der EGF'-R vermittelten Signalübertragung kann z.B. mit Zellen nachgewiesen werden, die humanen EGF-R exprimieren und deren Überleben und Proliferation von Stimulierung durch EGF bzw. TGF-alpha abhängt. Hier wurde eine Interleukin-3-(IL-3) abhängige Zellinie murinen Ursprungs verwendet, die derart genetisch verändert wurde, daß sie funktionellen humanen EGF-R exprimiert. Die Proliferation dieser F/L-HERc genannten Zellen kann daher entweder durch murines IL-3 oder durch EGF stimuliert werden (siehe von Rüden, T. et al. in EMBO J. 7, 2749-2756 (1988) und Pierce, J. H. et al. in Science 239, 628-631 (1988)).

Als Ausgangsmaterial für die F/L-HERc Zellen diente die Zelllinie FDC-P₁, deren Herstellung von Dexter, T. M. et al. in J. Exp. Med. 152, 1036-1047 (1980) beschrieben wurde. Alternativ können aber auch andere Wachstumsfaktor-abhängige Zellen verwendet werden (siehe beispielsweise Pierce, J. H. et al. in Science 239, 628-631 (1988), Shibuya, H. et al. in Cell 70, 57-67 (1992) und Alexander, W. S. et al. in EMBO J. 10, 3683-3691 (1991)). Zur Expression der humanen EGF-R cDNA (siehe Ullrich, A. et al. in Nature 309, 418-425 (1984)) wurden rekombinante Retroviren verwendet, wie in von Rüden, T. et al., EMBO J. 7, 2749-2756 (1988) beschrieben, mit dem Unterschied, daß zur Expression der EGF-R cDNA der retrovirale Vektor LXSN (siehe Miller, A. D. et al. in BioTechniques 7, 980-990 (1989)) eingesetzt wurde und als Verpackungszelle die Linie GP+E86 (siehe Markowitz, D. et al. in J. Virol. 62, 1120-1124 (1988)) diente.

Der Test wurde wie folgt durchgeführt:
F/L-HERc Zellen wurden in RPMI/1640 Medium (BioWhittaker), supplementiert mit '10 % foetalem Rinderserum (FCS, Boehringer Mannheim), 2 mM Glutamin (BioWhittaker), Standardantibiotika und 20 ng/ml humanem EGF (Promega), bei 37°C und 5% CO₂ kultiviert. Zur Untersuchung der inhibitorischen Aktivität der erfindungsgemäßen Verbindungen wurden 1,5 x 10⁴ Zellen pro Vertiefung in Triplikaten in 96-Loch-Platten in obigem Medium (200 µl) kultiviert, wobei die Proliferation der Zellen entweder mit EGF (20 ng/ml) oder murinem IL-3 stimuliert wurde. Als Quelle für IL-3 dienten Kulturüberstände der Zellinie X63/0 mIL-3 (siehe Karasuyama, H. et al.in Eur. J. Immunol. 18, 97-104 (1988)). Die erfindungsgemäßen Verbindungen wurden in 100% Dimethylsulfoxid (DMSO) gelöst und in verschiedenen Verdünnungen den Kulturen zugefügt, wobei die maximale DMSO Konzentration 1% betrug. Die Kulturen wurden für 48 Stunden bei 37°C inkubiert.

Zur Bestimmung der inhibitorischen Aktivität der erfindungsgemäßen Verbindungen wurde die relative Zellzahl mit dem Cell Titer 96^{™} AQᵤₑₒᵤₛ Non-Radioactive Cell Proliferation Assay (Promega) in O.D. Einheiten gemessen. Die relative Zellzahl wurde in Prozent der Kontrolle (F/LHERc Zellen ohne Inhibitor) berechnet und die Wirkstoffkonzentration, die die Proliferation der Zellen zu 50% hemmt (IC₅₀), abgeleitet. Hierbei wurden folgende Ergebnisse erhalten:

| Verbindung (Beispiel Nr.) | Hemmung der EGF-abhängigen Proliferation IC₅₀ [nM] |
|---|---|
| 1 | 5 |
| 1 (1) | 0.2 |

Die erfindungsgemäßen Verbindungen der allgemeinen Formel I hemmen somit die Signaltransduktion durch Tyrosinkinasen, wie am Beispiel des humanen EGF-Rezeptors gezeigt wurde, und sind daher nützlich zur Behandlung pathophysiologischer Prozesse, die durch Überfunktion von Tyrosinkinasen hervorgerufen werden. Das sind z.B. benigne oder maligne Tumoren, insbesondere Tumoren epithelialen und neuroepithelialen Ursprungs, Metastasierung sowie die abnorme Proliferation vaskulärer Endothelzellen (Neoangiogenese).

Die erfindungsgemäßen Verbindungen sind auch nützlich zur Vorbeugung und Behandlung von Erkrankungen der Atemwege und der Lunge, die mit einer vermehrten oder veränderten Schleimproduktion einhergehen, die durch Stimulation von Tyrosinkinasen hervorgerufen wird, wie z.B. bei entzündlichen Erkrankungen der Atemwege wie chronische Bronchitis, chronisch obstruktive Bronchitis, Asthma, Bronchiektasien, allergische oder nichtallergische Rhinitis oder Sinusitis, zystische Fibrose, α1-Antitrypsin-Mangel, oder bei Husten, Lungenemphysem, Lungenfibrose und hyperreaktiven Atemwegen.

Die Verbindungen sind auch geeignet für die Behandlung von Erkrankungen des Magen-Darm-Traktes und der Gallengänge und -blase, die mit einer gestörten Aktivität der Tyrosinkinasen einhergehen, wie sie z.B. bei chronisch entzündlichen Veränderungen zu finden sind, wie Cholezystitis, M. Crohn, Colitis ulcerosa, und Geschwüren im Magen-Darm-Trakt oder wie sie bei Erkrankungen des Magen-Darm-Traktes, die mit einer vermehrten Sekretion einhergehen, vorkommen, wie M. Ménétrier, sezernierende Adenome und Proteinverlustsyndrome.

Außerdem können die Verbindungen der allgemeinen Formel I und deren physiologisch verträglichen Salze zur Behandlung anderer Krankheiten verwendet werden, die durch aberrante Funktion von Tyrosinkinasen verursacht werden, wie z.B. epidermaler Hyperproliferation (Psoriasis), inflammatorischer Prozesse, Erkrankungen des Immunsystems, Hyperproliferation hämatopoetischer Zellen etc..

Auf Grund ihrer biologischen Eigenschaften können die erfindungsgemäßen Verbindungen allein oder in Kombination mit anderen pharmakologisch wirksamen Verbindungen angewendet werden, beispielsweise in der Tumortherapie in Monotherapie oder in Kombination mit anderen Anti-Tumor Therapeutika, beispielsweise in Kombination mit Topoisomerase-Inhibitoren (z.B. Etoposide), Mitoseinhibitoren (z.B. Vinblastin), mit Nukleinsäuren interagierenden Verbindungen (z.B. cis-Platin, Cyclophosphamid, Adriamycin), Hormon-Antagonisten (z.B. Tamoxifen), Inhibitoren metabolischer Prozesse (z.B. 5-FU etc.), Zytokinen (z.B. Interferonen), Antikörpern etc. Für die Behandlung von Atemwegserkrankungen können diese Verbindungen allein oder in Kombination mit anderen Atemwegstherapeutika, wie z.B. sekretolytisch, broncholytisch und/oder entzündungshemmend wirksamen Substanzen angewendet werden. Für die Behandlung von Erkrankungen im Bereich des Magen-Darm-Traktes können diese Verbindungen ebenfalls alleine oder in Kombination mit Motilitäts- oder Sekretions-beeinflussenden Substanzen gegeben werden. Diese Kombinationen können entweder simultan oder sequentiell verabreicht werden.

Die Anwendung dieser Verbindungen entweder alleine oder in Kombination mit anderen Wirkstoffen kann intravenös, subkutan, intramuskulär, intraperitoneal, intranasal, durch Inhalation oder transdermal oder oral erfolgen, wobei zur Inhalation insbesondere Aerosolformulierungen geeignet sind.

Bei der pharmazeutischen Anwendung werden die erfindungsgemäßen Verbindungen in der Regel bei warmblütigen Wirbeltieren, insbesondere beim Menschen, in Dosierungen von 0,01-100 mg/kg Körpergewicht, vorzugsweise bei 0,1-15 mg/kg verwendet. Zur Verabreichung werden diese mit einem oder mehreren üblichen inerten Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/- Sorbit, Wasser/Polyethylenglykol, Propylenglykol, Stearylalkohol, Carboxymethylcellulose oder fetthaltigen Substanzen wie Hartfett oder deren geeigneten Gemischen in übliche galenische Zubereitungen wie Tabletten, Dragees, Kapseln, Pulver, Suspensionen, Lösungen, Sprays oder Zäpfchen eingearbeitet.

Die nachfolgenden Beispiele sollen die vorliegende Erfindung näher erläutern ohne diese zu beschränken:
Herstellung der Ausgangsverbindungen:

### Beispiel I

### 6-Amino-4-[(3-bromphenyl)amino]-7-[3-(1-methyl-piperidin-4-yl)propyloxy]-chinazolin

1.00 g 4-[(3-Bromphenyl)amino]-7-[3-(1-methyl-piperidin-4-yl)-propyloxy]-6-nitro-chinazolin wird in 16 ml Wasser, 35 ml Ethanol und 1.3 ml Eisessig gelöst und zum Sieden erhitzt. Dann werden unter Rühren 540 mg Eisenpulver zugegeben. Das Reaktionsgemisch noch ca. 35 Minuten unter Rückfluß erhitzt. Zur Aufarbeitung wird das abgekühlte Reaktionsgemisch mit 15 ml Etanol verdünnt, mit 15N Natronlauge alkalisch gestellt, mit 20 g Extrelut versetzt und ca. 20 Minuten gerührt. Der entstandene Niederschlag wird abgesaugt und mit 200 ml warmem Ethanol nachgewaschen. Das Filtrat wird eingeengt, mit ca. 30 ml Wasser versetzt und 3 x mit je 70 ml Methylenchlorid/- Methanol (9:1) extrahiert. Die vereinigten Extrakte werden über Natriumsulfat getrocknet und eingeengt, wobei ein beigefarbener Feststoff zurückbleibt.
Ausbeute: 716 mg (76 % der Theorie)
Schmelzpunkt: 191-198°C
Massenspektrum (ESI⁺): m/z = 470, 472 [M+H]+

Analog Beispiel I werden folgende Verbindungen erhalten:
(1) 6-Amino-4-[(3-chlor-4-fluorphenyl)amino]-7-cyclopropyl-methoxy-chinazolin
   Schmelzpunkt: 209°C
   R_{f}-Wert: 0.68 (Kieselgel, Essigester)
(2) 6-Amino-4-[(3-chlor-4-fluorphenyl)amino]-7-cyclobutyloxy-chinazolin
   R_{f}-Wert: 0.32 (Kieselgel, Cyclohexan/Essigester = 3:4)
   Massenspektrum (ESI⁺). m/z = 359, 361 [M+H]⁺
(3) 6-Amino-4-[(3-chlor-4-fluorphenyl)amino]-7-cyclopentyloxy-chinazolin
   R_{f}-Wert: 0.33 (Kieselgel, Cyclohexan/Essigester = 1:1) Massenspektrum (ESI⁺) : m/z = 373, 375 [M+H] ⁺
(4) 6-Amino-4-[(R)-(1-phenyl-ethyl)amino]-7-cyclobutyloxy-chinazolin
   R_{f}-Wert: 0.28 (Kieselgel, Essigester)
   Massenspektrum (ESI⁺): m/z = 335 [M+H]⁺
(5) 6-Amino-4-[(*R*)-(1-phenyl-ethyl)amino]-7-cyclopropylmeth-oxy-chinazolin
   R_{f}-Wert: 0.54 (Kieselgel, Essigester)
   Massenspektrum (ESI⁺): m/z = 335 [M+H]⁺
(6) 6-Amino-4-[(R)-(1-phenyl-ethyl)amino]-7-cyclopentyloxy-chinazolin
   R_{f}-Wert: 0.20 (Kieselgel, Essigester)
   Massenspektrum (ESI⁺) : m/z = 349 [M+H] ⁺
(7) 6-Amino-4-[(3-chlor-4-fluorphenyl)amino]-7-[(tetrahydro-furan-2-yl)methoxy]-chinazolin
   Schmelzpunkt: 162-164°C
   R_{f}-Wert: 0.55 (Kieselgel, Essigester/Methanol=9:1)
   Massenspektrum (ESI⁻): m/z = 387, 389 [M-H]⁻
(8) 6-Amino-4-[(3-chlor-4-fluorphenyl)amino]-7-[(*S*)-(tetrahydrofuran-3-yl)oxy]-chinazolin
   R_{f}-Wert: 0.27 (Kieselgel, Essigester/Methanol=9:1)
   Massenspektrum (ESI⁻): m/z = 373, 375 [M-H]⁻
(9) 6-Amino-4-[(3-chlor-4-fluorphenyl)amino]-7-[(tetrahydropyran-4-yl)oxy]-chinazolin
   R_{f}-Wert: 0.41 (Kieselgel, Essigester/Methanol=9:1)
   Massenspektrum (ESI⁻): m/z = 387, 389 [M-H]⁻

### Beispiel II

### 4-[(3-Bromphenyl)amino]-7-[3-(1-methyl-piperidin-4-yl)propyl-oxy]-6-nitro-chinazblin

Zu einer Lösung aus 1.45 g 3-(1-Methyl-piperidin-4-yl)-propan-1-ol in 40 ml Tetrahydrofuran werden 360 mg Natriumhydrid gegeben. Die entstandene weiße Suspension wird 15 Minuten bei 65°C gerührt, abgekühlt und mit 1.45 g 4-[(3-Bromphenyl)amino]-7-fluor-6-nitro-chinazolin versetzt, wobei sich das Gemisch schlagartig dunkelrot färbt. Das Reaktionsgemisch wird zunächst noch 10 Minuten bei Raumtemperatur, anschließend 45 Minuten bei 65°C gerührt. Da die Umsetzung noch nicht vollständig ist, werden nochmals 150 mg Natriumhydrid zugesetzt und es wird weitere 45 Minuten bei 65°C gerührt. Das Lösungsmittel wird am Rotationsverdampfer abdestilliert und der braune Rückstand mit 50 ml Eiswasser verrührt. Die wäßrige Phase wird mit Methylenchlorid extrahiert. Die vereinigten Extrakte werden mit Wasser gewaschen, über Natriumsulfat getrocknet und eingeengt. Das Rohprodukt wird chromatographisch über eine Kieselgelsäule mit Methylenchlorid/Methanol/konzentrierter Ammoniak-Lösung (90:10:0.05) gereinigt.
Ausbeute: 1.30 g (65 % der Theorie)
R_{f}-Wert: 0.28 (Kieselgel, Methylenchlorid/Methanol/konzentrierte wäßrige Ammoniak-Lösung = 90:10:0.1)
Massenspektrum (ESI⁺): m/z = 500, 502 [M+H]⁺

Analog Beispiel II werden folgende Verbindungen erhalten:
(1) 4- [(3-Chlor-4-fluorphenyl)amino]-7-cyclopropylmethoxy-6-nitro-chinazolin (Durchführung in Dimethylformamid mit Kalium-tert.butylat als Base)
   Schmelzpunkt: 211-213°C
   Massenspektrum (ESI⁺): m/z = 389, 391 [M+H]⁺
(2) 4-[(3-Chlor-4-fluorphenyl)amino]-7-cyclobutyloxy-6-nitrochinazolin (Durchführung in Dimethylformamid mit Kalium-tert.-butylat als Base)
   Schmelzpunkt: 235°C
   R_{f}-Wert: 0.65 (Kieselgel, Cyclohexan/Essigester = 3:4)
(3) 4-[(3-Chlor-4-fluorphenyl)amino]-7-cyclopentyloxy-6-nitrochinazolin (Durchführung in Dimethylformamid mit Kalium-tert.-butylat als Base)
   Schmelzpunkt: 230°C
   Massenspektrum (ESI⁺): m/z = 403, 405 [M+H]⁺
(4) 4-[(R)-(1-Phenyl-ethyl)amino]-7-cyclobutyloxy-6-nitro-chinazolin (Durchführung in Dimethylformamid mit Kalium-tert.-butylat als Base)
   Schmelzpunkt: 108-110°C
   R_{f}-Wert: 0.54 (Kieselgel, Essigester)
(5) 4-[(R)-(1-Phenyl-ethyl)amino]-7-cyclopropylmethoxy-6-nitro-chinazolin (Durchführung in Dimethylformamid mit Kalium-tert.-butylat als Base)
   Schmelzpunkt: 155°C
   R_{f}-Wert: 0.24 (Kieselgel, Cyclohexan/Essigester = 1:1)
(6) 4-[(*R*)-(1-Phenyl-ethyl)amino]-7-cyclopentyloxy-6-nitro-chinazolin (Durchführung in Dimethylformamid mit Kalium-tert.-butylat als Base)
   R_{f}-Wert: 0.24 (Kieselgel, Petrolether/Essigester = 1:1)
   Massenspektrum (ESI⁺): m/z = 379 [M+H]⁺
(7) 4-[(3-Chlor-4-fluorphenyl)amino]-7-[(tetrahydrofuran-2-yl)methoxy]-6-nitro-chinazolin (Durchführung in Dimethylformamid mit Kalium'-tert.butylat als Base) R_{f}-Wert: 0.47 (Kieselgel, Essigester)
   Massenspektrum (ESI⁻): m/z = 417, 419 [M-H]⁻
(8) 4-[(3-Chlor-4-fluorphenyl)amino]-7-[(*S*)-(tetrahydrofuran-3-yl)oxy]-6-nitro-chinazolin (Durchführung in Dimethylformamid mit Kalium-tert.butylat als Base)
   R_{f}-Wert: 0.45 (Kieselgel, Essigester)
   Massenspektrum (ESI⁻): m/z = 403, 405 [M-H]⁻
(9) 4-[(3-Chlor-4-fluorphenyl)amino]-7-[(tetrahydropyran-4-yl)oxy]-6-nitro-chinazolin (Durchführung in Dimethylformamid mit Kalium-tert-butylat als Base)
   R_{f}-Wert: 0.41 (Kieselgel, Essigester)
   Massenspektrum (ESI⁻) : m/z = 417, 419 [M-H]⁻
(10) 4-[(R)-(1-Phenyl-ethyl)aminol-7-[2-(tetrahydropyran-2-yloxy)-ethoxy]-6-nitro-chinazolin
   R_{f}-Wert: 0.12 (Kieselgel, Cyclohexan/Essigester = 1:1)
   Massenspektrum (ESI⁺) : m/z = 439 [M+H]⁺
(11) 4-[(3-Chlor-4-fluorphenyl)amino]-7-{3-[(tert.butyl-dimethylsilyl)oxy]-propyloxy}-6-nitro-chinazolin (Durchführung in Dimethylformamid mit Kalium-tert-butylat als Base)
   R_{f}-Wert: 0.87 Kieselgel, Methylenchlorid/Methanol/konzentrierte
   wäßrige Ammoniak-Lösung = 90:10:0.1)
   Massenspektrum (ESI⁺) : m/z = 507, 509 [M+H]⁺

### Beispiel III

### 4-[(R)-(1-Phenyl-ethyl)amino]-6-nitro-7-fluor-chinazolin

Zu 108.8 g 4-Chlor-6-nitro-7-fluor-chinazolin in 800 ml Methylenchlorid wird 'eine Lösung von 74 ml (R)-1-Phenyl-ethyl-amin in 100 ml Dioxan unter Kühlung zugetropft Nach Rühren über Nacht bei Raumtemperatur wird mit Wasser ausgeschüttelt, die organische Phase wird getrocknet und eingeengt. Der Rückstand wird durch Chromatographie über eine Kieselgelsäule (Petrolether/Essigester = 1:1) gereinigt.
Ausbeute: 52.9 g (35% der Theorie),
Schmelzpunkt: 203°C
Massenspektrum (ESI⁺): m/z = 313 [M+H]⁺

### Beispiel IV

### 4-[(R)-(1-Phenyl-ethyl)amino]-7-[2-(methansulfonyloxy)-ethoxy]-6-nitro-chinazolin

Zu 8.08 g 4-[(R)-(1-Phenyl-ethyl)aminol-7-(2-hydroxy-ethoxy)-6-nitro-chinazolin und 4.53 ml Ethyldiisopropylamin in 90 ml Methylenchlorid wird unter Eisbad-Kühlung eine Lösung aus 1.79 ml Methansulfonsäurechlorid in 10 ml Methylenchlorid getropft. Das Reaktionsgemisch wird etwa eine Stunde bei Raumtemperatur gerührt, wobei insgeamt weitere 0.4 ml Methansulfonsäurechlorid und 0.5 ml Ethyldiisopropylamin nachgesetzt werden, bis die Umsetzung vollständig ist. Die Reaktionslösung wird mit Eiswasser und gesättigter Natriumcarbonat-Lösung verrührt, die organische Phase abgetrennt, mit Wasser gewaschen, über Magnesiumsulfat getrocknet und eingeengt. Der dunkle, harzartige Rückstand wird durch Verrühren mit wenig tert.Butylmethylether zur Kristallisation gebracht, abgesaugt und im Exsikkator getrocknet.
Ausbeute: 9,72 g (99 % der Theorie),
Schmelzpunkt: 128-134°C
Massenspektrum (ESI⁻) : m/z = 431 [M-H] ⁻

Analog Beispiel IV wird folgende Verbindung erhalten:
(1) 4-[(3-Chlor-4-fluorphenyl)amino]-7-[3-(methansulfonyloxy)-propyloxy]-6-nitro-chinazolin
   R_{f}-Wert: 0.75 (Kieselgel, Methylenchlorid/Methanol/konzentrierte wäßrige Ammoniak-Lösung = 90:10:0.1)
   Massenspektrum (ESI⁺): m/z = 471, 473 [M+H]⁺

### Beispiel V

### 4- [(R)-(1-Phenyl-ethyl)amino] -7- (2-,hydroxy-ethoxy)-6-nitro-chinazolin

Zu 8.05 g 4-[(R)-(1-Phenyl-ethyl)amino]-7-[2-(tetrahydropyran-2-yloxy)-ethoxy]-6-nitro-chinazolin in 120 ml Methanol werden 2 ml konzentrierte Salzsäure gegeben. Das Reaktionsgemisch wird 1.5 Stunden bei 50°C gerührt. Zur Aufarbeitung wird die Reaktionslösung mit gesättigter Natriumcarbonat-Lösung neutralisiert und eingeengt. Der feste Eindampfrückstand wird in Essigester aufgenommen. Die Lösung wird mit Wasser und gesättigter Natriumchlorid-Lösung gewaschen, über Magnesiumsulfat getrocknet und eingeengt. Der gelbliche Rückstand wird mit 20 ml tert.Butylmethylether verrührt, abgesaugt und im Exsikkator getrocknet.
Ausbeute: 4.53 g (91 % der Theorie),
Schmelzpunkt: 192-194°C
Massenspektrum (ESI⁻) : m/z = 353 [M-H]⁻

### Beispiel VI

### 4-[(3-Chlor-4-fluorphenyl)amino]-7-(3-hydroxy-propyloxy)-6-nitro-chinazolin

Hergestellt aus 4-[(3-Chlor-4-fluorphenyl)amino]-7-{3-[(tert.-butyldimethylsilyl)'oxy]-propyloxy}-6-nitro-chinazolin durch Abspaltung der Silylschutzgruppe mit Tetrabutylammoniumfluorid in Tetrahydrofuran.
Ausbeute: 94 % der Theorie,
R_{f}-Wert: 0.61 (Kieselgel, Methylenchlorid/Methanol/konzen-trierte wäßrige Ammoniak-Lösung = 90:10:0.1)
Massenspektrum (ESI⁻): m/z = 391, 393 [M-H]⁻

### Herstellung der Endprodukte:

### Beispiel 1

4-[(3-Chlor-4-fluorphenyl)amino]-6-{[4-(N,N-diethylamino)-1-oxo-2-buten-1-yl]amino}-7-cyclopropylmethoxy-chinazolin Zu einer Lösung aus 640 mg 4-Brom-2-butensäure in 10 ml Methylenchlorid werden bei Raumtemperatur 0.67 ml Oxalylchlorid und ein Tropfen Dimethylformamid gegeben Das Reaktionsgemisch wird noch ca. eine halbe Stunde bei Raumtemperatur gerührt, bis die Gasentwicklung beendet ist. Das entstandene Säurechlorid wird am Rotationsverdampfer im Vakuum weitgehend vom Lösungsmittel befreit. Anschließend wird das Rohprodukt in 10 ml Methylenchlorid gelöst und unter Eisbad-Kühlung zu einer Mischung aus 1.00 g 6-Amino-4-[(3-chlor-4-fluorphenyl)amino]-7-cyclopropylmethoxy-chinazolin und 1.60 ml Hünigbase in 50 ml Tetrahydrofuran getropft. Das Reaktionsgemisch wird 1.5 Stunden im Eisbad und weitere 2 Stunden bei Raumtemperatur gerührt. Dann werden 2.90 ml Diethylamin zugesetzt und das Gemisch wird 2.5 Tage bei Raumtemperatur gerührt. Zur Aufarbeitung wird das Reaktionsgemisch filtriert und das Filtrat eingeengt. Der Kolbenrückstand wird chromatographisch über eine Kieselgelsäule mit Essigester/Methanol (19:1) gereinigt.
Ausbeute: 550 mg (40 % der Theorie)
Schmelzpunkt: 114°C
Massenspektrum (ESI⁺) : m/z = 498, 500 [M+H] ⁺

Analog Beispiel 1 werden die folgenden Verbindungen erhalten:
(1) 4-[(3-Chlor-4-fluorphenyl)amino]-6-{[4-(morpholin-4-yl)-1-oxo-2-buten-1-yl]amino}-7-cyclopropylmethoxy-chinazolin R_{f}-Wert: 0.53 (Kieselgel, Essigester/Methanol = 9:1) Massenspektrum (ESI⁻): m/z = 510, 512 [M-H]⁻
(2) 4-[(3-Chlor-4-fluorphenyl)amino]-6-{[4-(4-ethyl-piperazin-1-yl)-1-oxo-2-buten-1-yl]amino}-7-cyclopropylmethoxy-chinazolin
   R_{f}-Wert: 0.44 (Kieselgel, Essigester/Methanol/konzentrierte
   wäßrige Ammoniak-Lösung = 9:1:0.1)
   Massenspektrum (EI): m/z = 538, 540 [M]⁺
(3) 4-[(3-Chlor-4-fluorphenyl)amino]-6-{[4-(2,6-dimethyl-morpholin-4-yl)-1-oxo-2-buten-1-yl]amino}-7-cyclopropylmethoxy-chinazolin
   Schmelzpunkt: 160°C
   Massenspektrum (ESI⁺): m/z = 540, 542 [M+H]⁺
(4) 4-[(3-Chlor-4-fluorphenyl)amino]-6-{[4-(dimethylamino)-1-oxo-2-buten-1-yl]amino}-7-cyclopropylmethoxy-chinazolin
   Schmelzpunkt: 137°C
   Massenspektrum (ESI⁺) : m/z = 470, 472 [M+H]⁺
(5) 4-[(3-Chlor-4-fluorphenyl)amino]-6-{[4-(morpholin-4-yl)-1-oxo-2-buten-1-yl]amino}-7-cyclobutyloxy-chinazolin
   R_{f}-Wert: 0.45 (Kieselgel, Essigester/Methanol = 9:1)
   Massenspektrum (ESI'⁺): m/z = 512, 514 [M+H]⁺
(6) 4-[(3-Chlor-4-fluorphenyl)amino]-6-{[4-(morpholin-4-yl)-1-oxo-2-buten-1-yl]aminol-7-cyclopentyloxy-chinazolin
   Schmelzpunkt: 143°C
   R_{f}-Wert: 0.45 (Kieselgel, Essigester/Methanol = 9:1)
(7) 4-[(3-Chlor-4-fluorphenyl)amino]-6-{[4-(diethylamino)-1-oxo-2-buten-1-yl]amino}-7-cyclobutyloxy-chinazolin
   Schmelzpunkt: 111°C
   R_{f}-Wert: 0.21 (Kieselgel, Essigester/Methanol = 9:1)
(8) 4-[(3-Chlor-4-fluorphenyl)amino]-6-{[4-(diethylamino)-1-oxo-2-buten-1-yl]amino}-7-cyclopentyloxy-chinazolin
   Schmelzpunkt: 105°C
   R_{f}-Wert: 0.23 (Kieselgel, Essigester/Methanol = 9:1)
(9) 4-[(R)-(1-Phenyl-ethyl)amino]-6-{[4-(morpholin-4-yl)-1-oxo-2-buten-1-yl]amino}-7-cyclobutyloxy-chinazolin
   R_{f}-Wert: 0.33 (Kieselgel, Essigester/Methanol = 9:1)
   Massenspektrum (ESI⁺) : m/z = 488 [M+H]⁺
(10) 4-[(R)-(1-Phenyl-ethyl)amino]-6-{[4-(morpholin-4-yl)-1-oxo-2-buten-1-yl]amino}-7-cyclopropylmethoxy-chinazolin
   R_{f}-Wert: 0.37 (Kieselgel, Essigester/Methanol = 9:1)
   Massenspektrum (ESI⁺) : m/z = 488 [M+H]⁺
(11) 4-[(R)-(1-Phenyl-ethyl)amino]-6-{[4-(morpholin-4-yl)-1-oxo-2-buten-1-yl]amino}-7-cyclopentyloxy-chinazolin
   R_{f}-Wert: 0.35 (Kieselgel, Essigester/Methanol = 9:1)
   Massenspektrum (ESI⁺) : m/z = 502 [M+H]⁺
(12) 4-[(R)-(1-Phenyl-ethyl)amino]-6-{[4-(diethylamino)-1-oxo-2-buten-1-yl]amino}-7-cyclobutyloxy-chinazolin
   R_{f}-Wert: 0.26 (Kieselgel, Essigester/Methanol = 4:1)
   Massenspektrum (ESI⁺): m/z = 474 [M+H]⁺
(13) 4-[(R)-(1-Phenyl-ethyl)amino]-6-[4-(diethylamino)-1-oxo-2-buten-1-yl]amino}-7-cyclopentyloxy-chinazolin
   R_{f}-Wert: 0.31 (Kieselgel, Essigester/Methanol = 4:1)
   Massenspektrum (ESI⁺): m/z = 488 [M+H]⁺
(14) 4-[(R)-(1-Phenyl-ethyl)amino]-6-{[4-(diethylamino)-1-oxo-2-buten-1-yl]amino}-7-cyclopropylmethoxy-chinazolin
   R_{f}-Wert: 0.15 (Kieselgel, Essigester/Methanol = 9:1)
   Massenspektrum (ESI⁺): m/z = 474 [M+H]⁺
(15) 4-[(3-Chlor-4-fluorphenyl)amino]-6-({4-[N-methyl-N-(1-methyl-piperidin-4-yl)-amino]-1-oxo-2-buten-1-yl}amino)-7-cyclopropylmethoxy-chinazolin
   R_{f}-Wert: 0.28 (Kieselgel, Essigester/Methanol/konz. wäßriges Ammoniak = 80:20:2)
   Massenspektrum (ESI⁺) : m/z = 553, 555 [M+H]⁺
(16) 4-[(3-Chlor-4-fluorphenyl)amino]-6-({4-[bis-(2-methoxyethyl)-amino]-1-oxo-2-buten-1-yl}amino)-7-cyclopropylmethoxy-chinazolin
   R_{f}-Wert: 0.51 (Kieselgel, Essigester/Methanol = 9:1)
   Massenspektrum (ESI⁺): m/z = 558, 560 [M+H]⁺
(17) 4-[(3-Chlor-4-fluorphenyl)amino]-6-({4-[N-ethyl-N-(2-methoxyethyl)-amino]-1-oxo-2-buten-1-yl}amino)-7-cyclopropylmethoxy-chinazolin
   R_{f}-Wert: 0.33 (Kieselgel, Essigester/Methanol = 9:1)
   Massenspektrum (ESI⁺): m/z = 528, 530 [M+H]⁺
(18) 4-[(3-Chlor-4-fluorphenyl)amino]-6-{[4-(piperidin-1-yl)-1-oxo-2-buten-1-yl]amino}-7-cyclopropylmethoxy-chinazolin
   R_{f}-Wert: 0.22 (Kieselgel, Essigester/Methanol = 9:1)
   Massenspektrum (ESI⁺): m/z = 510, 512 [M+H]⁺
(19) 4-[(3-Chlor-4-fluorphenyl)amino]-6-{[4-(2-methyl-piperidin-1-yl)-1-oxo-2-buten-1-yl]amino}-7-cyclopropylmethoxy-chinazolin
   R_{f}-Wert: 0.21 (Kieselgel, Essigester/Methanol = 9:1)
   Massenspektrum (ESI⁺): m/z = 524, 526 [M+H]⁺
(20) 4-[(3-Chlor-4-fluorphenyl)amino]-6-{[4-(pyrrolidin-1-yl)-1-oxo-2-buten-1-yl]amino}-7-cyclopropylmethoxy-chinazolin
   R_{f}-Wert: 0.10 (Kieselgel, Essigester/Methanol = 9:1)
   Massenspektrum (ESI⁺): m/z = 496, 498 [M+H]⁺
(21) 4-[(3-Chlor-4-fluorphenyl)amino]-6-{[4-(2-methyl-pyrrolidin-1-yl)-1-oxo-2-buten-1-yl]amino}-7-cyclopropylmethoxy-chinazolin
   Schmelzpunkt: 108-110°C
   R_{f}-Wert: 0.27 (Kieselgel, Essigester/Methanol = 9:1)
(22) 4-[(3-Chlor-4-fluorphenyl)amino]-6-{[4-(cis-2,6-dimethyl-piperidin-1-yl)-1-oxo-2-buten-1-yl}amino]-7-cyclopropylmethoxy-chinazolin
   R_{f}-Wert: 0.27 (Kieselgel, Essigester/Methanol = 9:1)
   Massenspektrum (ESI⁻) : m/z = 536, 538 [M-H]⁻
(23) 4-[(3-Chlor-4-fluorphenyl)amino]-6-{[4-(2,5-dimethyl-pyrrolidin-1-yl)-1-oxo-2-buten-1-yl]amino}-7-cyclopropyl-methoxy-chinazolin
   R_{f}-Wert: 0.36 (Kieselgel, Essigester/Methanol = 9:1)
   Massenspektrum (ESI⁻): m/z = 522, 524 [M-H]⁻
(24) 4-[(3-Chlor-4-fluorphenyl)amino]-6-{[4-(diethylamino)-1-oxo-2-buten-1-yl]amino}-7-[(tetrahydrofuran-2-yl)methoxy]-chinazolin
   R_{f}-Wert: 0.35 (Kieselgel, Essigester/Methanol/konz. wässriges Ammoniak = 9:1:0.1)
   Massenspektrum (ESI⁻) : m/z - 526, 528 [M-H]⁻
(25) 4-[(3-Chlor-4-fluorphenyl)amino]-6-{[4-(diethylamino)-1-oxo-2-buten-1-yl]amino}-7-[(*S*)-(tetrahydrofuran-3-yl)oxy]-chinazolin
   Schmelzpunkt: 119°C
   Massenspektrum (ESI⁻): m/z = 512, 514 [M-H]⁻
(26) 4-[(3-Chlor-4-fluorphenyl)amino]-6-{[4-(4-diethylamino-methyl-piperidin-1-yl)-1-oxo-2-buten-1-yl]amino}-7-cyclo-propylmethoxy-chinazolin
   R_{f}-Wert: 0.20 (Kieselgel, Methylenchlorid/Methanol = 9:1) Massenspektrum (ESI⁻): m/z = 593, 595 [M-H]⁻
(27) 4-[(3-Chlor-4-fluorphenyl)amino]-6-{[4-(N-methyl-N-cyclo-propylmethyl-amino)-1-oxo-2-buten-1-yl]amino}-7-cyclopropyl-methoxy-chinazolin
   R_{f}-Wert: 0.73 (Kieselgel, Methylenchlorid/Methanol = 9:1)
   Massenspektrum (ESI⁺) : m/z = 510, 512 [M+H]⁺
(28) 4-[(3-Chlor-4-'fluorphenyl)amino]-6-({4-[N-methyl-N-(2-methoxypropyl)-amino]-1-oxo-2-buten-1-yl}amino)-7-cyclopropylmethoxy-chinazolin (Das eingesetzte N-Methyl-N-(2-methoxypropyl)-amin wurde durch Umsetzung von 2-Methoxy-propionsäurechlorid mit Methylamin und anschließender Reduktion mit Lithiumaluminiumhydrid erhalten)
   Schmelzpunkt: 123-125°C
   R_{f}-Wert: 0.66 (Kieselgel, Methylenchlorid/Methanol = 9:1)
(29) 4-[(3-Chlor-4-fluorphenyl)amino]-6-({4-[N-methyl-N-(3-methoxypropyl)-amino]-1-oxo-2-buten-1-yl}amino)-7-cyclopropylmethoxy-chinazolin
   R_{f}-Wert: 0.66 (Kieselgel, Methylenchlorid/Methanol = 9:1)
   Massenspektrum (ESI⁺): m/z = 528, 530 [M+H] ⁺
(30) 4-[(3-Chlor-4-fluorphenyl)amino]-6-{[4-(diethylamino)-1-oxo-2-buten-1-yl]amino}-7-[(tetrahydropyran-4-yl)oxy]-chinazolin
   R_{f}-Wert: 0.47 (Kieselgel, Methylenchlorid/Methanol = 9:1)
   Massenspektrum (ESI⁻) : m/z = 528, 530 [M-H]⁻
(31) 4-[(3-Chlor-4-fluorphenyl)amino]-6-({4-[N-methyl-N-(1-methoxy-2-propyl)-amino]-1-oxo-2-buten-1-yl}amino)-7-cyclopropylmethoxy-chinazolin
   (Das eingesetzte N-Methyl-N-(1-methoxy-2-propyl)-amin wurde durch reduktive Aminierung von Methoxyaceton mit Methylaminhydrochlorid und Natriumtriacetoxyborhydrid in Gegenwart von Natriumacetat erhalten. Die Reaktion wurde in Tetrahydrofuran durchgeführt.)
   R_{f}-Wert: 0.38 (Kieselgel, Essigester/Methanol = 9:1)
   Massenspektrum (ESI⁺): m/z = 528, 530 [M+H]⁺

Analog den vorstehenden Beispielen und anderen literaturbekannten Verfahren können auch die folgenden Verbindungen erhalten werden:
(1) 4-[(3-Chlor-4-fluorphenyl)amino]-6-{[4-(N,N-dimethyl-amino)-1-oxo-2-buten-1-yl]amino}-7-cyclopropylmethoxy-chinazolin
(2) 4-[(3-Chlor-4-fluorphenyl)amino]-6-{[4-(piperidin-1-yl)-1-oxo-2-buten-1-yl]amino}-7-cyclopropylmethoxy-chinazolin
(3) 4-[(3-Chlor-4-fluorphenyl)amino]-6-{[4-(2,6-dimethyl-morpholin-4-yl)-1-oxo-2-buten-1-yl]amino}-7-cyclopropylmethoxy-chinazolin
(4) 4-[(3-Chlor-4-fluorphenyl)amino]-6-{[4-(pyrrolidin-1-yl)-1-oxo-2-buten-1-yl]amino}-7-cyclopropylmethoxy-chinazolin
(5) 4-[(3-Chlor-4-fluorphenyl)amino]-6-{[4-(N-cyclopropyl-N-methylamino)-1-oxo-2-buten-1-yl]amino}-7-cyclopropylmethoxy-chinazolin
(6) 4-[(3-Chlor-4-fluorphenyl)amino]-6-{[4-(N-cyclopropylmethyl-N-methylamino)-1-oxo-2-buten-1-yl]amino}-7-cyclopropylmethoxy-chinazolin
(7) 4-[(3-Chlor-4-fluorphenyl)amino]-6-{[4-(N,N-dimethylamino)-1oxo-2-butin-1yl]amino}-7-cyclopropylmethoxy-chinazolin
(8) 4-[(3-Chlor-4-fluorphenyl)amino]-6-{[4-(N,N-diethylamino)-1-oxo-2-butin-1-yl]amino}-7-cyclopropylmethoxy-chinazolin
(9) 4-[(3-Chlor-4-fluorphenyl)amino]-6-{[4-(piperidin-1yl)-1-oxo-2-butin-1-yl]amino}-7-cyclopropylmethoxy-chinazolin
(10) 4-[(3-Chlor-4-fluorphenyl)amino]-6-{[4-(morpholin-4-yl)-1-oxo-2-butin-1-yl]amino}-7-cyclopropylmethoxy-chinazolin
(11) 4-[(3-Chlor-4-fluorphenyl)amino]-6-({4-[N,N-bis(2-methoxy-ethyl)amino]-1-oxo-2-buten-1-yl}amino)-7-cyclopropylmethoxy-chinazolin
(12) 4-[(3-Chlor-4-fluorphenyl)amino]-6-({4-[N-(2-methoxy-ethyl)-N-methylamino]-1-oxo-2-buten-1-yl}amino)-7-cyclopropylmethoxy-chinazolin
(13) 4-[(3-Chlor-4-fluorphenyl)amino]-6-({4-[bis-(2-methoxy-ethyl)-amino]-1-oxo-2-buten-1-yl}amino)-7-cyclobutyloxy-chinazolin
(14) 4-[(3-Chlor-4-fluorphenyl)amino]-6-({4-[N-methyl-N-(2-methoxyethyl)-amino]-1-oxo-2-buten-1-yl}amino)-7-cyclo-butyloxy-chinazolin
(15) 4-[(3-Chlor-4-fluorphenyl)amino]-6-({4-[(S-N-methyl-N-(1-methoxy-2-propyl)-ami.no]-1-oxo-2-buten-1-yl}amino)-7-cyclobutyloxy-chinazolin
(16) 4-[(3-Chlor-4-fluorphenyl)amino]-6-({4-[(R)-N-methyl-N-(1-methoxy-2-propyl)-amino]-1-oxo-2-buten-1-yl}amino)-7-cyclobutyloxy-chinazolin
(17) 4-[(3-Chlor-4-fluorphenyl)amino]-6-({4-[N-methyl-N-(1-methoxy-2-propyl)-amino]-1-oxo-2-buten-1-yl}amino)-7-cyclopropylmethoxy-chinazolin
(18) 4-[(3-Chlor-4-fluorphenyl)amino]-6-({4-[N-methyl-N-(2-methoxypropyl)-amino]-1-oxo-2-buten-1-yl}amino)-7-cyclopropylmethoxy-chinazolin
(19) 4-[(3-Chlor-4-fluorphenyl)amino]-6-({4-[N-methyl-N-(3-methoxypropyl)-amino]-1-oxo-2-buten-1-yl}amino)-7-cyclopropylmethoxy-chinazolin
(20) 4-[(3-Chlor-4-fluorphenyl)amino]-6-{[4-(N-cyclopropyl-N-methyl-amino)-1-oxo-2-buten-1-yl]amino}-7-cyclobutyloxy-chinazolin
(21) 4-[(3-Chlor-4-fluorphenyl)amino]-6-{[4-(N-cyclopropylmethyl-N-methyl-amino)-1-oxo-2-buten-1-yl]amino}-7-cyclobutyloxy-chinazolin
(22) 4-[(3-Chlor-4-fluorphenyl)amino]-6-{[4-(pyrrolidin-1-yl)-1-oxo-2-buten-1-yl]amino}-7-cyclobutyloxy-chinazolin
(23) 4-[(3-Chlor-4-fluorphenyl)amino]-6-{[4-(2-methyl-pyrrolidin-1-yl)-1-oxo-2-buten-1-yl]amino}-7-cyclobutyloxy-chinazolin
(24) 4-[(3-Chlor-4-fluorphenyl)amino]-6-{[4-(2,5-dimethyl-pyrrolidin-1-yl)-1-oxo-2-buten-1-yl]amino}-7-cyclobutyloxy-chinazolin
(25) 4-[(3-Chlor-4-fluorphenyl)amino]-6-{[4-(piperidin-1-yl)-1-oxo-2-buten-1-yl]amino}-7-cyclobutyloxy-chinazolin
(26) 4-[(3-Chlor-4-fluorphenyl)amino]-6-{[4-(2-methyl-piperidin-1-yl)-1-oxo-2-buten-1-yl] amino}-7-cyclobutyloxy-chinazolin
(27) 4-[(3-Chlor-4-fluorphenyl)amino]-6-{[4-(2,6-dimethyl-piperidin-1-yl)-1-oxo-2-buten-1-yl]amino}-7-cyclobutyloxy-chinazolin
(28) 4-[(3-Chlor-4-fluorphenyl)amino}-6-{[4-(3-methyl-morpholin-4-yl)-1-oxo-2-buten-1-yl]amino}-7-cyclobutyloxy-chinazolin
(29) 4-[(3-Chlor-4-fluorphenyl)amino]-6-{[4-(3,5-dimethyl-morpholin-4-yl)-1-oxo-2-buten-1-yl]amino}-7-cyclobutyloxy-chinazolin
(30) 4-[(3-Chlor-4-fluorphenyl)amino]-6-({4-[N-methyl-N-(2-methoxyethyl)-amino]-1-oxo-2-buten-1-yl}amino)-7-(tetrahydrofuran-3-yl-oxy)-chinazolin
(31) 4-[(3-Chlor-4-fluorphenyl)amino]-6-({4-[N-methyl-N-(2-methoxyethyl)-amino]-1-oxo-2-buten-1-yl}amino)-7-(tetrahydropyran-4-yl-oxy)-chinazolin
(32) 4-[(3-Chlor-4-fluorphenyl)amino]-6-({4-[N-methyl-N-(2-methoxyethyl)-amino]-1-oxo-2-buten-1-yl}amino)-7-(tetrahydrofuran-2-yl-methoxy)-chinazolin

### Beispiel 2

### Dragées mit 75 mg Wirksubstanz

| 1 Dragéekern enthält: | |
|---|---|
| Wirksubstanz | 75,0 mg |
| Calciumphosphat ' | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1,5 mg |
| | 230,0 mg |

### Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm-Maschenweite gerieben und mit der restlichen Menge Magnesiumstearat vermischt. Dieses Granulat wird auf einer Tablettiermaschine zu Tabletten mit der gewünschten Form gepreßt.
Kerngewicht: 230 mg
Stempel: 9 mm, gewölbt

Die so hergestellten Dragéekerne werden mit einem Film überzogen, der im wesentlichen aus Hydroxypropylmethylcellulose besteht. Die fertigen Filmdragees werden mit Bienenwachs geglänzt.
Dragéegewicht: 245 mg.

### Beispiel 3

### Tabletten mit 100 mg Wirksubstanz

### Zusammensetzung:

| 1 Tablette enthält: | |
|---|---|
| Wirksubstanz | 100,0 mg |
| Milchzucker | 80,0 mg |
| Maisstärke | 34,0 mg |
| Polyvinylpyrrolidon | 4,0 mg |
| Magnesiumstearat | 2,0 mg |
| | 220,0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet.
Tablettengewicht: 220 mg
Durchmesser: 10 mm, biplan mit beidseitiger Facette und einseitiger Teilkerbe.

### Beispiel 4

### Tabletten mit 150 mg Wirksubstanz

### Zusammensetzung:

| 1 Tablette enthält: | |
|---|---|
| Wirksubstanz | 150,0 mg |
| Milchzucker pulv. | 89,0 mg |
| Maisstärke | 40,0 mg |
| Kolloide Kieselgelsäure | 10,0 mg |
| Polyvinylpyrrolidon | 10,0 mg |
| Magnesiumstearat | 1,0 mg |
| | 300,0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen.

Das bei 45°C getrocknete Granulat wird nochmals durch dasselbe Sieb gerieben und mit der angegebenen Menge Magnesiumstearat gemischt. Aus der Mischung werden Tabletten gepreßt.
Tablettengewicht: 300 mg
Stempel: 10 mm, flach

### Beispiel 5

### Hartgelatine-Kapseln mit 150 mg Wirksubstanz

| 1 Kapsel enthält: | | |
|---|---|---|
| Wirkstoff | | 150,0 mg |
| Maisstärke getr. | ca. | 180,0 mg |
| Milchzucker pulv. | ca. | 87,0 mg |
| Magnesiumstearat | | 3,0 mg |
| | ca. | 420,0 mg |

### Herstellung:

Der Wirkstoff wird mit den Hilfsstoffen vermengt, durch ein Sieb von 0,75 mm-Maschenweite gegeben und in einem geeigneten Gerät homogen gemischt.

Die Endmischung wird in Hartgelatine-Kapseln der Größe 1 abgefüllt.
Kapselfüllung: ca. '320 mg
Kapselhülle: Hartgelatine-Kapsel Größe 1.

### Beispiel 6

### Suppositorien mit 1'50 mg Wirksubstanz

| 1 Zäpfchen enthält: | |
|---|---|
| Wirkstoff | 150,0 mg |
| Polyäthylenglykol 1500 | 550,0 mg |
| Polyäthylenglykol 6000 | 460,0 mg |
| Polyoxyäthylensorbitanmonostearat | 840,0 mg |
| | 2 000,0 mg |

### Herstellung:

Nach dem Aufschmelzen der Suppositorienmasse wird der Wirkstoff darin homogen verteilt und die Schmelze in vorgekühlte Formen gegossen.

### Beispiel 7

### Suspension mit 50 mg Wirksubstanz

| 100 ml Suspension enthalten: | |
|---|---|
| Wirkstoff | 1,00 g |
| Carboxymethylcellulose-Na-Salz | 0,10 g |
| p-Hydroxybenzoesäuremethylester | 0,05 g |
| p-Hydroxybenzoesäurepropylester | 0,01 g |
| Rohrzucker | 10,00 g |
| Glycerin | 5,00 g |
| Sorbitlösung 70%ig | 20,00 g |
| Aroma | 0,30 g |
| Wasser dest. ad | 100 ml |

### Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.
5 ml Suspension enthalten 50 mg Wirkstoff.

### Beispiel 8

### Ampullen mit 10 mg Wirksubstanz

### Zusammensetzung:

| | | |
|---|---|---|
| Wirkstoff | | 10,0 mg |
| 0,01 n Salzsäure s.q. | | |
| Aqua bidest | ad | 2,0 ml |

### Herstellung:

Die Wirksubstanz wird in der erforderlichen Menge 0,01 n HCl gelöst, mit Kochsalz isotonisch gestellt, sterilfiltriert und in 2 ml Ampullen abgefüllt.

### Beispiel 9

### Ampullen mit 50 mg Wirksubstanz

### Zusammensetzung:

| | |
|---|---|
| Wirkstoff | 50,0 mg |
| 0,01 n Salzsäure s.q. | |
| Aqua bidesta | ad 10,0 ml |

### Herstellung:

Die Wirksubstanz wird in der erforderlichen Menge 0,01 n HCl gelöst, mit Kochsalz isotonisch gestellt, sterilfiltriert und in 10 ml Ampullen abgefüllt.

### Beispiel 10

### Kapseln zur Pulverinhalation mit 5 mg Wirksubstanz

### 1 Kapsel enthält:

| | |
|---|---|
| Wirksubstanz | 5,0 mg |
| Lactose für Inhalationszwecke | 15,0 mg |
| | 20,0 mg |

### Herstellung:

Die Wirksubstanz wird mit Lactose für Inhalationszwecke gemischt. Die Mischung wird auf einer Kapselmaschine in Kapseln (Gewicht der Leerkapsel ca. 50 mg) abgefüllt.
Kapselgewicht: 70,0 mg
Kapselgröße: 3

### Beispiel 11

### Inhalationslösung für Handvernebler mit 2,5 mg Wirksubstanz

### 1 Hub enthält:

| | |
|---|---|
| Wirksubstanz | 2,500 mg |
| Benzalkoniumchlorid | 0,001 mg |
| 1N-Salzsäure q.s. | |
| Ethanol/Wasser (50/50) | ad 15,000 mg |

### Herstellung:

Die Wirksubstanz und Benzalkoniumchlorid werden in Ethanol/- Wasser (50/50) gelöst. Der pH-Wert der Lösung wird mit 1N-Salzsäure eingestellt. Die eingestellte Lösung wird filtriert und in für den Handvernebler geeignete Behälter (Kartuschen) abgefüllt.
Füllmasse des Behälters: 4,5 g

## Patentansprüche

1. Bicycliche Heterocyclen der allgemeinen Formel 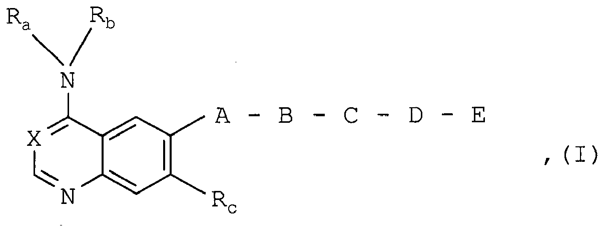 in der
Rₐ ein Wasserstoffatom,
R_{b} eine 1-Phenylethylgruppe oder eine Phenylgruppe, in der der Phenylkern durch die Reste R₁ und R₂ substituiert ist, wobei
R₁ und R₂, die gleich oder verschieden sein können, jeweils ein Wasserstoff-, Fluor-, Chlor- oder Bromatom darstellen,
X ein Stickstoffatom,
A eine Iminogruppe,
B eine Carbonylgruppe,
C eine 1,2-Vinylen- oder Ethinylengruppe,
D eine Methylengruppe,
E eine Dimethylamino-, Diethylamino-, Bis-(2-methoxy-ethyl)-amino-, N-Methyl-N-(2-methoxy-ethyl)-amino-, N-Ethyl-N-(2-methoxy-ethyl)-amino-, N-Methyl-N-cyclopropyl-amino-, N-Methyl-N-cyclopropylmethyl-amino-, N-Methyl-N-(1-methoxy-2-propyl)-amino-, N-Methyl-N-(2-methoxy-propyl)-amino- oder N-Methyl-N-(3-methoxy-propyl)-amino-gruppe,
eine gegebenenfalls durch eine oder zwei Methylgruppen substituierte Pyrrolidino-, Piperidino- oder Morpholinogruppe, und
R_{c} eine Cyclopropylmethoxy-, Cyclobutyloxy- oder Cyclopentyloxygruppe,
eine Tetrahydrofuran-3-yloxy-, Tetrahydropyran-4-yloxy- oder Tetrahydrofuran-2-ylmethoxygruppe bedeuten,
deren Tautomere, deren Stereoisomere und deren Salze.

2. Folgende Verbindungen der allgemeinen Formel I gemäß Anspruch 1:
(1) 4-[(3-Chlor-4-fluorphenyl)amino]-6-{[4-(N,N-diethylamino)-1-oxo-2-buten-1-yl]amino}-7-cyclopropylmethoxy-chinazolin,
(2) 4-[(3-Chlor-4-fluorphenyl)amino]-6-{[4-(morpholin-4-yl)-1-oxo-2-buten-1-yl]amino}-7-Cyclopropylmethoxy-chinazolin,
(3) 4-[(3-Chlor-4-fluorphenyl)amino]-6-{[4-(2,6-dimethyl-morpholin-4-yl)-1-oxo-2-buten-1-yl]amino}-7-cyclopropylmethoxy-chinazolin,
(4) 4-[(3-Chlor-4-fluorphenyl)amino]-6-{[4-(dimethylamino)-1-oxo-2-buten-1-yl]amino}-7-cyclopropylmethoxy-chinazolin,
(5) 4-[(3-Chlor-4-fluorphenyl)amino]-6-{[4-(morpholin-4-yl)-1-oxo-2-buten-1-yl]amino}-7-cyclobutyloxy-chinazolin,
(6) 4-[(3-Chlor-4-fluorphenyl)amino]-6-{[4-(morpholin-4-yl)-1-oxo-2-buten-1-yl]amino}-7-cyclopentyloxy-chinazolin,
(7) 4-[(3-Chlor-4-fluorphenyl)amino]-6-{[4-(diethylamino)-1-oxo-2-buten-1-yl]amino}-7-Cyclobutyloxy-chinazolin,
(8) 4-[(3-Chlor-4-fluorphenyl)amino]-6-{[4-(diethylamino)-1-oxo-2-buten-1-yl]amino}-7-cyclopentyloxy-chinazolin,
(9) 4-[(R)-(1-Phenyl-ethyl)amino]-6-{[4-(morpholin-4-yl)-1-oxo-2-buten-1-yl]amino}-7-cyclobutyloxy-chinazolin,
(10) 4-[(R)-(1-Phenyl-ethyl)amino]-6-{[4-(morpholin-4-yl)-1-oxo-2-buten-1-yl]amino}-7-cyclopropylmethoxy-chinazolin,
(11) 4-[(R)-(1-Phenyl-ethyl)aminol-6-{[4-(morpholin-4-yl)-1-oxo-2-buten-1-yl]amino}-7-cyclopentyloxy-chinazolin,
(12) 4-[(R)-(1-Phenyl-ethyl)amino]-6-{[4-(diethylamino)-1-oxo-2-buten-1-yl]amino}-7-Cyclobutyloxy-chinazolin,
(13) 4-[(R)-(1-Phenyl-ethyl)amino]-6-{[4-(diethylamino)-1-oxo-2-buten-1-yl]amino}-7-cyclopentyloxy-chinazolin,
(14) 4-[(R)-(1-Phenyl-ethyl)amino]-6-{[4-(diethylamino)-1-oxo-2-buten-1-yl]amino}-7-cyclopropylmethoxy-chinazolin,
(15) 4-[(3-Chlor-4-fluorphenyl)amino]-6-({4-[bis-(2-methoxy-ethyl)-amino]-1-oxo-2-buten-1-yl}amino)-7-cyclopropylmethoxy-chinazolin,
(16) 4-[(3-Chlor-4-fluorphenyl)amino]-6-({4-[N-ethyl-N-(2-methoxyethyl)-amino]-1-oxo-2-buten-1-yl}amino)-7-cyclopropyl-methoxy-chinazolin,
(17) 4-[(3-Chlor-4-fluorphenyl)amino]-6-{[4-(piperidin-1-yl)-1-oxo-2-buten-1-yl]amino}-7-cyclopropylmethoxy-chinazolin,
(18) 4-[(3-Chlor-4-fluorphenyl)amino]-6-{[4-(2-methyl-piperidin-1-yl)-1-oxo-2-buten-1-yl]amino}-7-cyclopropylmethoxy-chinazolin,
(19) 4-[(3-Chlor-4-fluorphenyl)amino]-6-{[4-(pyrrolidin-1-yl) - 1-oxo-2-buten-1-yl]amino}-7-cyclopropylmethoxy-chinazolin,
(20) 4-[(3-Chlor-4-fluorphenyl)amino]-6-{[4-(2-methyl-pyrrolidin-1-yl)-1-oxo-2-buten-1-yl]amino}-7-cyclopropylmethoxy-chinazolin,
(21) 4-[(3-Chlor-4-fluorphenyl)amino]-6-{[4-(cis-2,6-dimethyl-piperidin-1-yl)-1-oxo-2-buten-1-yl]amino}-7-cyclopropylmethoxy-chinazolin,
(22) 4-[(3-Chlor-4-fluorphenyl)amino]-6-{[4-(2,5-dimethyl-pyrrolidin-1-yl)-1-oxo-2-buten-1-yl]amino}-7-cyclopropyl-methoxy-chinazolin,
(23) 4-[(3-Chlor-4-fluorphenyl)amino]-6-{[4-(diethylamino)-1-oxo-2-buten-1-yl]amino}-7-[(tetrahydrofuran-2-yl)methoxy]-chinazolin,
(24) 4-[(3-Chlor-4-fluorphenyl)amino]-6-{[4-(diethylamino)-1-oxo-2-buten-1-yl]amino}-7-[(S)-(tetrahydrofuran-3-yl)oxy]-chinazolin,
(25) 4-[(3-Chlor-4-fluorphenyl)amino]-6-{[4-(4-diethylamino-methyl-piperidin-1-yl)-1-oxo-2-buten-1-yl]amino}-7-cyclo-propylmethoxy-chinazolin,
(26) 4-[(3-Chlor-4-fluorphenyl)amino]-6-{[4-(N-methyl-N-cyclo-propylmethyl-amino)-1-oxo-2-buten-1-yl]amino}-7-cyclopropylmethoxy-chinazolin,
(27) 4-[(3-Chlor-4-fluorphenyl)amino]-6-({4-[N-methyl-N-(2-methoxypropyl)-amino]-1-oxo-2-buten-1-yl}amino)-7-cyclopropylmethoxy-chinazolin,
(28) 4-[(3-Chlor-4-fluorphenyl)amino]-6-({4-[N-methyl-N-(3-methoxypropyl)-amino]-1-oxo-2-buten-1-yl}amino)-7-cyclopropylmethoxy-chinazolin,
(29) 4-[(3-Chlor-4-fluorphenyl)amino]-6-{[4-(diethylamino)-1-oxo-2-buten-1-yl]amino}-7-[(tetrahydropyran-4-yl)oxy]-chinazolin,
(30) 4-[(3-Chlor-4-fluorphenyl)amino]-6-({4-[N-methyl-N-(1-methoxy-2-propyl)-amino]-1-oxo-2-buten-1-yl}amino)-7-cyclopropylmethoxy-chinazolin,
(31) 4-[(3-Chlor-4-fluorphenyl)amino]-6-{[4-(N,N-dimethylamino)-1-oxo-2-butin-1-yl]amino}-7-Cyclopropylmethoxy-chinazolin,
(32) 4-[(3-Chlor-4-fluorphenyl)amino]-6-{[4-(N,N-diethylamino)-1-oxo-2-butin-1-yl]amino}-7-cyclopropylmethoxy-chinazolin,
(33) 4-[(3-Chlor-4-fluorphenyl)amino]-6-{[4-(piperidin-1-yl)-1-oxo-2-butin-1-yl]amino}-7-cyclopropylmethoxy-chinazolin,
(34) 4-[(3-Chlor-4-fluorphenyl)amino]-6-{[4-(morpholin-4-yl)-1-oxo-2-butin-1-yl]amino}-7-cyclopropylmethoxy-chinazolin,
(35) 4-[(3-Chlor-4-fluorphenyl)amino]-6-{[4-(N,N-dimethylamino)-1-oxo-2-buten-1-yl]amino}-7-(2-cyclopropyl-ethoxy)-chinazolin,
(36) 4-[(3-Chlor-4-fluorphenyl)amino]-6-({4-[bis-(2-methoxy-ethyl)-amino]-1-oxo-2-buten-1-yl}amino)-7-cyclobutyloxy-chinazolin,
(37) 4-[(3-Chlor-4-fluorphenyl)amino]-6-({4-[N-methyl-N-(2-methoxyethyl)-amino]-1-oxo-2-buten-1-yl}amino)-7-cyclobutyloxy-chinazolin,
(38) 4-[(3-Chlor-4-fluorphenyl)amino]-6-({4-[(S)-N-methyl-N-(1-methoxy-2-propyl)-amino]-1-oxo-2-buten-1-yl}amino)-7-cyclobutyloxy-chinazolin,
(39) 4-[(3-Chlor-4-fluorphenyl)amino]-6-({4-[(R)-N-methyl-N-(1-methoxy-2-propyl)-amino]-1-oxo-2-buten-1-yl}amino)-7-cyclobutyloxy-chinazolin,
(40) 4-[(3-Chlor-4-fluorphenyl)amino]-6-({4-[N-methyl-N-(1-methoxy-2-propyl)-amino]-1-oxo-2-buten-1-yl}amino)-7-cyclopropylmethoxy-chinazolin,
(41) 4-[(3-Chlor-4-fluorphenyl)amino]-6-({4-[N-methyl-N-(2-methoxypropyl)-amino]-1-oxo-2-buten-1-yl}amino)-7-cyclopropylmethoxy-chinazolin,
(42) 4-[(3-Chlor-4-fluorphenyl)amino]-6-({4-[N-methyl-N-(3-methoxypropyl)-amino]-1-oxo-2-buten-1-yl}amino)-7-cyclopropyl-methoxy-chinazolin,
(43) 4-[(3-Chlor-4-fluorphenyl)amino]-6-{[4-(N-cyclopropyl-N-methyl-amino)-1-oxo-2-buten-1-yl]amino}-7-cyclobutyloxy-chinazolin,
(44) 4-[(3-Chlor-4-fluorphenyl)amino]-6-{[4-(N-cyclopropylmethyl-N-methyl-amino)-1-oxo-2-buten-1-yl]amino}-7-cyclobutyloxy-chinazolin,
(45) 4-[(3-Chlor-4-fluorphenyl)amino]-6-{[4-(pyrrolidin-1-yl)-1-oxo-2-buten-1-yllaminol-7-cyclobutyloxy-chinazolin,
(46) 4-[(3-Chlor-4-fluorphenyl)amino]-6-{[4-(2-methyl-pyrrolidin-1-yl)-1-oxo-2-buten-1-yl]amino}-7-cyclobutyloxy-chinazolin,
(47) 4-[(3-Chlor-4-fluorphenyl)amino]-6-{[4-(2,5-dimethyl-pyrrolidin-1-yl)-1-oxo-2-buten-1-yl]amino}-7-cyclobutyloxy-chinazolin,
(48) 4-[(3-Chlor-4-fluorphenyl)amino]-6-{[4-(piperidin-1-yl) - 1-oxo-2-buten-1-yl]amino}-7-cyclobutyloxy-chinazolin,
(49) 4-[(3-Chlor-4-fluorphenyl)amino]-6-{[4-(2-methyl-piperidin-1-yl)-1-oxo-2-buten-1-yl]amino}-7-cyclobutyloxy-chinazolin,
(50) 4-[(3-Chlor-4-fluorphenyl)amino]-6-{[4-(2,6-dimethyl-piperidin-1-yl)-1-oxo-2-buten-1-yl]amino}-7-cyclobutyloxy-chinazolin,
(51) 4-[(3-Chlor-4-fluorphenyl)amino]-6-{[4-(3-methyl-morpholin-4-yl)-1-oxo-2-buten-1-yl]amino}-7-cyclobutyloxy-chinazolin,
(52) 4-[(3-Chlor-4-fluorphenyl)amino]-6-{[4-(3,5-dimethyl-morpholin-4-yl)-1-oxo-2-buten-1-yl]amino}-7-cyclobutyloxy-chinazolin,
(53) 4-[(3-Chlor-4-fluorphenyl)amino]-6-({4-[N-methyl-N-(2-methoxyethyl)-amino]-1-oxo-2-buten-1-yl}amino)-7-(tetrahydrofuran-3-yl-oxy)-chinazolin,
(54) 4-[(3-Chlor-4-fluorphenyl)amino]-6-({4-[N-methyl-N-(2-methoxyethyl)-amino]-1-oxo-2-buten-1-yl}amino)-7-(tetrahydropyran-4-yl-oxy)-chinazolin und
(55) 4-[(3-Chlor-4-fluorphenyl)amino]-6-({4-[N-methyl-N-(2-methoxyethyl)-amino]-1-oxo-2-buten-1-yl}amino)-7-(tetrahydrofuran-2-yl-methoxy)-chinazolin,
sowie deren Salze.

3. Physiologisch verträgliche Salze der Verbindungen nach Anspruch 1 oder 2 mit anorganischen oder organischen Säuren oder Basen.

4. Arzneimittel, enthaltend eine Verbindung nach Anspruch 1 oder 2 oder ein physiologisch verträgliches Salz gemäß Anspruch 3 neben gegebenenfalls einem oder mehreren inerten Trägerstoffen und/oder Verdünnungsmitteln.

5. Verwendung einer Verbindung nach mindestens einem der Ansprüche 1 bis 3 zur Herstellung eines Arzneimittels, das zur Behandlung von benignen oder malignen Tumoren, zur Vorbeugung und Behandlung von Erkrankungen der Atemwege und der Lunge sowie zur Behandlung von Erkrankungen des Magen-Darm-Traktes und der Gallengänge und -blase geeignet ist.

6. Verfahren zur Herstellung eines Arzneimittels gemäß Anspruch 4, **dadurch gekennzeichnet, daß** auf nichtchemischem Wege eine Verbindung nach mindestens einem der Ansprüche 1 bis 3 in einen oder mehrere inerte Trägerstoffe und/oder Verdünnungsmittel eingearbeitet wird.

7. Verfahren zur Herstellung der Verbindungen der allgemeinen Formel I gemäß den Ansprüchen 1 bis 3, **dadurch gekennzeichnet, daß**
a) eine Verbindung der allgemeinen Formel 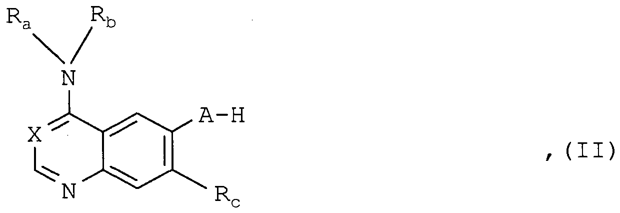 in der
Rₐ bis R_{c}, A und X wie in den Ansprüchen 1 oder 2 erwähnt definiert sind, mit einer Verbindung der allgemeinen Formel
Z₁ - B - C - D - E , (III)
in der
B bis E wie in den Ansprüchen 1 oder 2 erwähnt definiert sind und
Z₁ eine Austrittsgruppe darstellt, umgesetzt wird oder
b) zur Herstellung von Verbindungen der allgemeinen Formel I, in der der Rest E über ein Stickstoffatom mit dem Rest D verknüpft ist, eine Verbindung der allgemeinen Formel 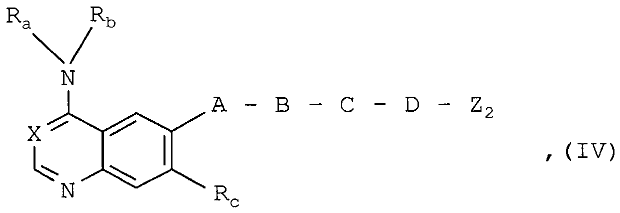 in der
Rₐ bis R_{c}, A bis D und X wie in den Ansprüchen oder 2 erwähnt definiert sind und
Z₂ eine Austrittsgruppe darstellt, mit einer Verbindung der allgemeinen Formel
H - E' , (V)
in der
E' einen der für E in den Ansprüchen 1 oder 2 erwähnten Reste darstellt, der über ein Stickstoffatom mit dem Rest D verknüpft ist, umgesetzt wird und
gewünschtenfalls eine so erhaltene Verbindung der allgemeinen Formel I, die eine Amino-, Alkylamino- oder Iminogruppe enthält, mittels Acylierung oder Sulfonylierung in eine entsprechende Acyl- oder Sulfonylverbindung der allgemeinen Formel I übergeführt wird und/oder
eine so erhaltene Verbindung der allgemeinen Formel I, die eine Amino-, Alkylamino- oder Iminogruppe enthält, mittels Alkylierung oder reduktiver Alkylierung in eine entsprechende Alkylverbindung der allgemeinen Formel I übergeführt wird und/oder eine so erhaltene Verbindung der allgemeinen Formel I, die eine Carboxy- oder Hydroxyphosphorylgruppe enthält, mittels Veresterung in einen entsprechenden Ester der allgemeinen Formel I übergeführt wird und/oder
eine so erhaltene Verbindung der allgemeinen Formel I, die eine Carboxy- oder Estergruppe enthält, mittels Umsetzung mit einem entsprechenden Amin in ein entsprechendes Amid der allgemeinen Formel I übergeführt wird und/oder
erforderlichenfalls ein bei den vorstehend beschriebenen Umsetzungen verwendeter Schutzrest wieder abgespalten wird und/oder
gewünschtenfalls eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträgliche Salze übergeführt wird.

## Claims

1. Bicyclic heterocycles of general formula 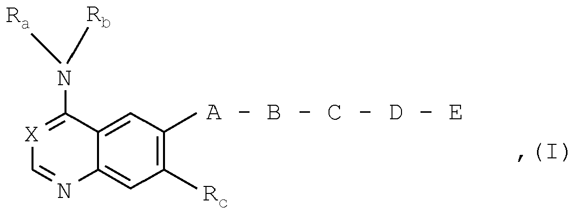 wherein
Rₐ denotes a hydrogen atom,
R_{b} denotes a 1-phenylethyl group or a phenyl group wherein the phenyl nucleus is substituted by the groups R₁ and R₂, whilst
R₁ and R₂, which may be identical or different, in each case denote a hydrogen, fluorine, chlorine or bromine atom,
X denotes a nitrogen atom,
A denotes an imino group,
B denotes a carbonyl group,
C denotes a 1,2-vinylene or ethynylene group,
D denotes a methylene group,
E denotes a dimethylamino, diethylamino, bis-(2-methoxy-ethyl)-amino, N-methyl-N-(2-methoxy-ethyl)-amino, N-ethyl-N-(2-methoxy-ethyl)-amino, N-methyl-N-cyclopropyl-amino, N-methyl-N-cyclopropylmethyl-amino, N-methyl-N-(1-methoxy-2-propyl)-amino, N-methyl-N-(2-methoxy-propyl)-amino or N-methyl-N-(3-methoxy-propyl)-amino group,
a pyrrolidino, piperidino or morpholino group each optionally substituted by one or two methyl groups and
R_{c} denotes a cyclopropylmethoxy, cyclobutyloxy or cyclopentyloxy group,
a tetrahydrofuran-3-yloxy, tetrahydropyran-4-yloxy or tetra-hydrofuran-2-ylmethoxy group,
the tautomers, stereoisomers and salts thereof.

2. The following compounds of general formula I according to claim 1:
(1) 4-[(3-chloro-4-fluorophenyl)amino]-6-{[4-(N,N-diethylamino)-1-oxo-2-buten-1-yl]amino}-7-cyclopropylmethoxy-quinazoline,
(2) 4-[(3-chloro-4-fluorophenyl)amino]-6-{[4-(morpholin-4-yl)-1-oxo-2-buten-1-yl]amino}-7-cyclopropylmethoxy-quinazoline,
(3) 4-[(3-chloro-4-fluorophenyl)amino]-6-{[4-(2,6-dimethyl-morpholin-4-yl)-1-oxo-2-buten-1-yl]amino}-7-cyclopropylmethoxy-quinazoline,
(4) 4-[(3-chloro-4-fluorophenyl)amino]-6-{[4-(dimethylamino)-1-oxo-2-buten-1-yl]amino}-7-cyclopropylmethoxy-quinazoline,
(5) 4-[(3-chloro-4-fluorophenyl)amino]-6-{[4-(morpholin-4-yl)-1-oxo-2-buten-1-yl]amino}-7-cyclobutyloxy-quinazoline,
(6) 4-[(3-chloro-4-fluorophenyl)amino]-6-{[4-(morpholin-4-yl)-1-oxo-2-buten-1-yl]amino}-7-cyclopentyloxy-quinazoline,
(7) 4-[(3-chloro-4-fluorophenyl)amino]-6-{[4-(diethylamino)-1-oxo-2-buten-1-yl]amino}-7-cyclobutyloxy-quinazoline,
(8) 4-[(3-chloro-4-fluorophenyl)amino]-6-{[4-(diethylamino)-1-oxo-2-buten-1-yl]amino}-7-cyclopentyloxy-quinazoline,
(9) 4-[(*R*)-(1-phenyl-ethyl)amino]-6-{[4-(morpholin-4-yl)-1-oxo-2-buten-1-yl]amino}-7-cyclobutyloxy-quinazoline,
(10) 4-[(*R*)-(1-phenyl-ethyl)amino]-6-{[4-(morpholin-4-yl)-1-oxo-2-buten-1-yl]amino}-7-cyclopropylmethoxy-quinazoline,
(11) 4-[(*R*)-(1-phenyl-ethyl)amino]-6-{[4-(morpholin-4-yl)-1-oxo-2-buten-1-yl]amino}-7-cyclopentyloxy-quinazoline,
(12) 4-[(*R*)-(1-phenyl-ethyl)amino]-6-{[4-(diethylamino)-1-oxo-2-buten-1-yl]amino}-7-cyclobutyloxy-quinazoline,
(13) 4-[(*R*)-(1-phenyl-ethyl)amino]-6-{[4-(diethylamino)-1-oxo-2-buten-1-yl]amino}-7-cyclopentyloxy-quinazoline,
(14) 4-[(*R*)-(1-phenyl-ethyl)amino]-6-{[4-(diethylamino)-1-oxo-2-buten-1-yl]amino}-7-cyclopropylmethoxy-quinazoline,
(15) 4-[(3-chloro-4-fluorophenyl)amino]-6-({4-[bis-(2-methoxyethyl)-amino]-1-oxo-2-buten-1-yl}amino)-7-cyclopropylmethoxy-quinazoline,
(16) 4-[(3-chloro-4-fluorophenyl)amino]-6-({4-[N-ethyl-N-(2-methoxyethyl)-amino]-1-oxo-2-buten-1-yl}amino)-7-cyclopropylmethoxy-quinazoline,
(17) 4-[(3-chloro-4-fluorophenyl)amino]-6-{[4-(piperidin-1-yl)-1-oxo-2-buten-1-yl]amino}-7-cyclopropylmethoxy-quinazoline,
(18) 4-[(3-chloro-4-fluorophenyl)amino]-6-{[4-(2-methyl-piperidin-1-yl)-1-oxo-2-buten-1-yl]amino}-7-cyclopropylmethoxy-quinazoline,
(19) 4-[(3-chloro-4-fluorophenyl)amino]-6-{[4-(pyrrolidin-1-yl)-1-oxo-2-buten-1-yl]amino}-7-cyclopropylmethoxy-quinazoline,
(20) 4-[(3-chloro-4-fluorophenyl)amino]-6-{[4-(2-methyl-pyrrolidin-1-yl)-1-oxo-2-buten-1-yl]amino}-7-cyclopropylmethoxy-quinazoline,
(21) 4-[(3-chloro-4-fluorophenyl)amino]-6-{[4-(cis-2,6-dimethyl-piperidin-1-yl)-1-oxo-2-buten-1-yl]amino}-7-cyclopropylmethoxy-quinazoline,
(22) 4-[(3-chloro-4-fluorophenyl)amino]-6-{[4-(2,5-dimethyl-pyrrolidin-1-yl)-1-oxo-2-buten-1-yl]amino}-7-cyclopropylmethoxy-quinazoline,
(23) 4-[(3-chloro-4-fluorophenyl)amino]-6-{[4-(diethylamino)-1-oxo-2-buten-1-yl]amino}-7-[(tetrahydrofuran-2-yl)methoxy]-quinazoline,
(24) 4-[(3-chloro-4-fluorophenyl)amino]-6-([4-(diethylamino)-1-oxo-2-buten-1-yl]amino}-7-[(S)-(tetrahydrofuran-3-yl)oxy]-quinazoline,
(25) 4-[(3-chloro-4-fluorophenyl)amino]-6-{[4-(4-diethylaminomethyl-piperidin-1-yl)-1-oxo-2-buten-1-yl]amino}-7-cyclopropylmethoxy-quinazoline,
(26) 4-[(3-chloro-4-fluorophenyl)amino]-6-{[4-(N-methyl-N-cyclopropylmethyl-amino)-1-oxo-2-buten-1-yl]amino}-7-cyclopropylmethoxy-quinazoline,
(27) 4-[(3-chloro-4-fluorophenyl)amino]-6-({4-[N-methyl-N-(2-methoxypropyl)-amino]-1-oxo-2-buten-1-yl}amino)-7-cyclopropylmethoxy-quinazoline,
(28) 4-[(3-chloro-4-fluorophenyl)amino]-6-({4-[N-methyl-N-(3-methoxypropyl)-amino]-1-oxo-2-buten-1-yl}amino)-7-cyclopropylmethoxy-quinazoline,
(29) 4-[(3-chloro-4-fluorophenyl)amino]-6-{[4-(diethylamino)-1-oxo-2-buten-1-yl]amino}-7-[(tetrahydropyran-4-yl)oxy]-quinazoline,
(30) 4-[(3-chloro-4-fluorophenyl)amino]-6-({4-[N-methyl-N-(1-methoxy-2-propyl)-amino]-1-oxo-2-buten-1-yl}amino)-7-cyclopropylmethoxy-quinazoline,
(31) 4-[(3-chloro-4-fluorophenyl)amino]-6-{[4-(N,N-dimethylamino)-1-oxo-2-butyn-1-yl]amino}-7-cyclopropylmethoxy-quinazoline,
(32) 4-[(3-chloro-4-fluorophenyl)amino]-6-{[4-(N,N-diethylamino)-1-oxo-2-butyn-1-yl]amino}-7-cyclopropylmethoxy-quinazoline,
(33) 4-[(3-chloro-4-fluorophenyl)amino]-6-{[4-(piperidin-1-yl)-1-oxo-2-butyn-1-yl]amino}-7-cyclopropylmethoxy-quinazoline,
(34) 4-[(3-chloro-4-fluorophenyl)amino]-6-{[4-(morpholin-4-yl)-1-oxo-2-butyn-1-yl]amino}-7-cyclopropylmethoxy-quinazoline,
(35) 4-[(3-chloro-4-fluorophenyl)amino]-6-{[4-(N,N-dimethylamino)-1-oxo-2-buten-1-yl]amino}-7-(2-cyclopropyl-ethoxy)-quinazoline,
(36) 4-[(3-chloro-4-fluorophenyl)amino]-6-({4-[bis-(2-methoxyethyl)-amino]-1-oxo-2-buten-1-yl}amino)-7-cyclobutyloxy-quinazoline,
(37) 4-[(3-chloro-4-fluorophenyl)amino]-6-({4-[N-methyl-N-(2-methoxyethyl)-amino]-1-oxo-2-buten-1-yl}amino)-7-cyclobutyloxy-quinazoline,
(38) 4-[(3-chloro-4-fluorophenyl)amino]-6-({4-[(*S*)-N-methyl-N-(1-methoxy-2-propyl)-amino]-1-oxo-2-buten-1-yl}amino)-7-cyclobutyloxy-quinazoline,
(39) 4-[(3-chloro-4-fluorophenyl)amino]-6-({4-[(*R*)-N-methyl-N-(1-methoxy-2-propyl)-amino]-1-oxo-2-buten-1-yl}amino)-7-cyclobutyloxy-quinazoline,
(40) 4-[(3-chloro-4-fluorophenyl)amino]-6-({4-[N-methyl-N-(1-methoxy-2-propyl)-amino]-1-oxo-2-buten-1-yl}amino)-7-cyclopropylmethoxy-quinazoline,
(41) 4-[(3-chloro-4-fluorophenyl)amino]-6-({4-[N-methyl-N-(2-methoxypropyl)-amino]-1-oxo-2-buten-1-yl}amino)-7-cyclopropylmethoxy-quinazoline,
(42) 4-[(3-chloro-4-fluorophenyl)amino]-6-({4-[N-methyl-N-(3-methoxypropyl)-amino]-1-oxo-2-buten-1-yl}amino)-7-cyclopropylmethoxy-quinazoline,
(43) 4-[(3-chloro-4-fluorophenyl)amino]-6-{[4-(N-cyclopropyl-N-methyl-amino)-1-oxo-2-buten-1-yl]amino}-7-cyclobutyloxy-quinazoline,
(44) 4-[(3-chloro-4-fluorophenyl)amino]-6-{[4-(N-cyclopropylmethyl-N-methyl-amino)-1-oxo-2-buten-1-yl]amino}-7-cyclobutyloxy-quinazoline,
(45) 4-[(3-chloro-4-fluorophenyl)amino]-6-{[4-(pyrrolidin-1-yl)-1-oxo-2-buten-1-yl]amino}-7-cyclobutyloxy-quinazoline,
(46) 4-[(3-chloro-4-fluorophenyl)amino]-6-{[4-(2-methyl-pyrrolidin-1-yl)-1-oxo-2-buten-1-yl]amino}-7-cyclobutyloxy-quinazoline,
(47) 4-[(3-chloro-4-fluorophenyl)amino]-6-{[4-(2,5-dimethyl-pyrrolidin-1-yl)-1-oxo-2-buten-1-yl]amino}-7-cyclobutyloxy-quinazoline,
(48) 4-[(3-chloro-4-fluorophenyl)amino]-6-{[4-(piperidin-1-yl)-1-oxo-2-buten-1-yl]amino}-7-cyclobutyloxy-quinazoline,
(49) 4-[(3-chloro-4-fluorophenyl)amino]-6-{[4-(2-methyl-piperidin-1-yl)-1-oxo-2-buten-1-yl]amino}-7-cyclobutyloxy-quinazoline,
(50) 4-[(3-chloro-4-fluorophenyl)amino]-6-{[4-(2,6-dimethyl-piperidin-1-yl)-1-oxo-2-buten-1-yl]amino}-7-cyclobutyloxy-quinazoline,
(51) 4-[(3-chloro-4-fluorophenyl)amino]-6-{[4-(3-methyl-morpholin-4-yl)-1-oxo-2-buten-1-yl]amino}-7-cyclobutyloxy-quinazoline,
(52) 4-[(3-chloro-4-fluorophenyl)amino]-6-{[4-(3,5-dimethyl-morpholin-4-yl)-1-oxo-2-buten-1-yl]amino}-7-cyclobutyloxy-quinazoline,
(53) 4-[(3-chloro-4-fluorophenyl)amino]-6-({4-[N-methyl-N-(2-methoxyethyl)-amino]-1-oxo-2-buten-1-yl}amino)-7-(tetrahydrofuran-3-yl-oxy)-quinazoline,
(54) 4-[(3-chloro-4-fluorophenyl)amino]-6-({4-[N-methyl-N-(2-methoxyethyl)-amino]-1-oxo-2-buten-1-yl}amino)-7-(tetrahydropyran-4-yl-oxy)-quinazoline, and
(55) 4-[(3-chloro-4-fluorophenyl)amino]-6-({4-[N-methyl-N-(2-methoxyethyl)-amino]-1-oxo-2-buten-1-yl}amino)-7-(tetrahydrofuran-2-yl-methoxy)-quinazoline, as well as the salts thereof.

3. Physiologically acceptable salts of the compounds according to claim 1 or 2 with inorganic or organic acids or bases.

4. Medicaments containing a compound according to claim 1 or 2 or a physiologically acceptable salt according to claim 3 optionally together with one or more inert carriers and/or diluents.

5. Use of a compound according to at least one of claims 1 to 3 for preparing a medicament which is suitable for treating benign or malignant tumours, for preventing and treating diseases of the airways and lungs and for treating diseases of the gastrointestinal tract and the bile duct and gall bladder.

6. Process for preparing a medicament according to claim 4, **characterised in that** a compound according to at least one of claims 1 to 3 is incorporated in one or more inert carriers and/or diluents by a non-chemical method.

7. Process for preparing the compounds of general formula I according to claims 1 to 3, **characterised in that**
a) a compound of general formula 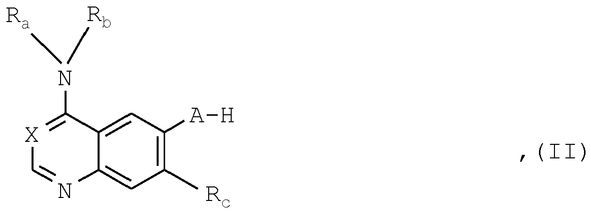 wherein
Rₐ to R_{c}, A and X are defined as in claims 1 or 2, is reacted with a compound of general formula
Z₁-B-C-D-E , (III)
wherein
B to E are defined as in claims 1 or 2 and
Z₁ denotes a leaving group, or
b) in order to prepare compounds of general formula I wherein the group E is linked to the group D via a nitrogen atom, a compound of general formula 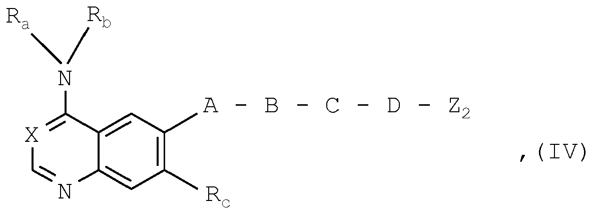 wherein
Rₐ to R_{c}, A to D and X are defined as in claims 1 or 2 and
Z₂ denotes a leaving group, is reacted with a compound of general formula
H-E' , (V)
wherein
E' denotes one of the groups mentioned for E in claims 1 or 2 which is linked to the group D via a nitrogen atom, and
if desired a compound of general formula I thus obtained which contains an amino, alkylamino or imino group is converted by acylation or sulphonylation into a corresponding acyl or sulphonyl compound of general formula I, and/or
a compound of general formula I thus obtained which contains an amino, alkylamino or imino group is converted by alkylation or reductive alkylation into a corresponding alkyl compound of general formula I, and/or
a compound of general formula I thus obtained which contains a carboxy or hydroxyphosphoryl group is converted by esterification into a corresponding ester of general formula I, and/or
a compound of general formula I thus obtained which contains a carboxy or ester group is converted by reaction with a corresponding amine into a corresponding amide of general formula I, and/or
if necessary any protecting group used during the reactions described hereinbefore is cleaved again, and/or
if desired a compound of general formula I thus obtained is resolved into its stereoisomers, and/or
a compound of general formula I thus obtained is converted into the salts thereof, more particularly, for pharmaceutical use, into the physiologically acceptable salts thereof.

## Revendications

1. Hétérocycles bicycliques de formule générale 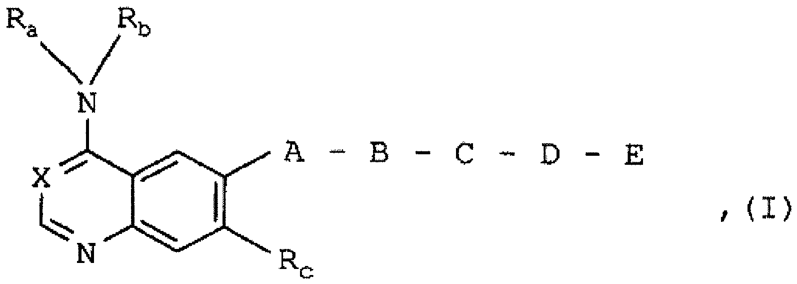 dans laquelle
Rₐ désigne un atome d'hydrogène,
R_{b} désigne un groupe 1-phényléthyle ou un groupe phényle dans lequel le noyau phényle est substitué par les radicaux R₁ et R₂, où
R₁ et R₂ qui peuvent être identiques ou différents, représentent respectivement un atome d'hydrogène, de fluor, de chlore ou de brome,
X désigne un atome d'azote,
A désigne un groupe imino,
B désigne un groupe carbonyle,
C désigne un groupe 1,2-vinylène ou éthinylène,
D désigne un groupe méthylène,
E désigne un groupe diméthylmino, diéthylamino, bis-(2-méthoxy-éthyl)-amino, N-méthyl-N-(2-méthoxy-éthyl)-amino, N-éthyl-N-(2-méthoxy-éthyl)-amino, N-méthyl-N-cyclopropyl-amino, N-méthyl-N-cyclopropylméthyl-amino, N-méthyl-N-(1-méthoxy-2-propyl)-amino, N-méthyl-N-(2-méthoxy-propyl)-amino ou N-méthyl-N-(3-méthoxy-propyl)amino,
un groupe pyrrolidino, pipéridino ou morpholino substitué éventuellement par un ou deux groupes méthyle, et
R_{c} désigne un groupe cyclopropylméthoxy, cyclobutyloxy ou cyclopentyloxy,
un groupe tétrahydrofuran-3-yloxy, tétrahydropyran-4-yloxy ou tétrahydrofuran-2-ylméthoxy,
leurs tautomères, leurs stéréo-isomères et leurs sels.

2. Composés suivants de formule générale I selon la revendication 1 :
(1) 4-[(3-chloro-4-fluorophényl)amino]-6-{[4-(N,N-diéthylamino)-1-oxo-2-butén-1-yl]amino}-7-cyclopropylméthoxy-quinazoline,
(2) 4-[(3-chloro-4-fluorophényl)amino]-6-{[4-(morpholin-4-yl)-1-oxo-2-butén-1-yl]amino}-7-cyclopropylméthoxy-quinazoline,
(3) 4-[(3-chloro-4-fluorophényl)amino]-6-{[4-(2,6-diméthyl-morpholin-4-yl)-1-oxo-2-butén-1-yl]amino}-7-cyclopropylméthoxy-quinazoline,
(4) 4-[(3-chloro-4-fluorophényl)amino]-6-{[4-(diméthylamino)-1-oxo-2-butén-1-yl]amino}-7-cyclopropylméthoxy-quinazoline,
(5) 4-[(3-chloro-4-fluorophényl)amino]-6-{[4-(morpholin-4-yl)-1-oxo-2-butén-1-yl]amino}-7-cyclobutyloxy-quinazoline,
(6) 4-[(3-chloro-4-fluorophényl)amino]-6-{[4-(morpholin-4-yl)-1-oxo-2-butén-1-yl]amino}-7-cyclopentyloxy-quinazoline,
(7) 4-[(3-chloro-4-fluorophényl)amino]-6-{[4-(diéthylamino)-1-oxo-2-butén-1-yl]amino}-7-cyclobutyloxy-quinazoline,
(8) 4-[(3-chloro-4-fluorophényl)amino]-6-{[4-(diéthylamino)-1-oxo-2-butén-1-yl]amino}-7-cyclopentyloxy-quinazoline,
(9) 4-[(R)(1-phényl-éthyl)amino]-6-{[4-(morpholin-4-yl)-1-oxo-2-butén-1-yl]amino}-7-cyclobutyloxy-quinazoline,
(10) 4-[(R)(1-phényl-éthyl)amino]-6-{[4-(morpholin-4-yl)-1-oxo-2-butén-1-yl]amino}-7-cyclopropylméthoxy-quinazoline,
(11) 4-[(R)(1-phényl-éthyl)amino]-6-{[4-(morpholin-4-yl)-1-oxo-2-butén-1-yl]amino}-7-cyclopentyloxy-quinazoline,
(12) 4-[(R)(1-phényl-éthyl)amino]-6-{[4-(diéthylamino)-1-oxo-2-butén-1-yl]amino}-7-cyclobutyloxy-quinazoline,
(13) 4-[(R)(1-phényl-éthyl)amino]-6-{[4-(diéthylamino)-1-oxo-2-butén-1-yl]amino}-7-cyclopentyloxy-quinazoline,
(14) 4-[(R)(1-phényl-éthyl)amino]-6-{[4-(diéthylamino)-1-oxo-2-butén-1-yl]amino}-7-cyclopropylméthoxy-quinazoline,
(15) 4-[(3-chloro-4-fluorophényl)amino]-6-({4-[bis-(2-méthoxy-éthyl)-amino]-1-oxo-2-butén-1-yl}amino)-7-cyclopropylméthoxy-quinazoline,
(16) 4-[(3-chloro-4-fluorophényl)amino]-6-({4-[N-éthyl-N-(2-méthoxy-éthyl)-amino]-1-oxo-2-butén-1-yl}amino)-7-cyclopropylméthoxy-quinazoline,
(17) 4-[(3-chloro-4-fluorophényl)amino]-6-{[4-(pipéridin-1-yl)-1-oxo-2-butén-1-yl]amino}-7-cyclopropylméthoxy-quinazoline,
(18) 4-[(3-chloro-4-fluorophényl)amino]-6-{[4-(2-méthyl-pipéridin-1-yl)-1-oxo-2-butén-1-yl]amino}-7-cyclopropylméthoxy-quinazoline,
(19) 4-[(3-chloro-4-fluorophényl)amino]-6-{[4-(pyrrolidin-1-yl)-1-oxo-2-butén-1-yl]amino}-7-cyclopropylméthoxy-quinazoline,
(20) 4-[(3-chloro-4-fluorophényl)amino]-6-{[4-(2-méthyl-pyrrolidin-1-yl)-1-oxo-2-butén-1-yl]amino}-7-cyclopropylméthoxy-quinazoline,
(21) 4-[(3-chloro-4-fluorophényl)amino]-6-{[4-(cis-2,6-diméthyl-pipéridin-1-yl)-1-oxo-2-butén-1-yl]amino}-7-cyclopropylméthoxy-quinazoline,
(22) 4-[(3-chloro-4-fluorophényl)amino]-6-{[4-(2,5-diméthyl-pyrrolidin-1-yl)-1-oxo-2-butén-1-yl]amino}-7-cyclopropylméthoxy-quinazoline,
(23) 4-[(3-chloro-4-fluorophényl)amino]-6-{[4-(diéthylamino)-1-oxo-2-butén-1-yl]amino}-7-[(tétrahydrofuran-2-yl)méthoxy]-quinazoline,
(24) 4-[(3-chloro-4-fluorophényl)amino]-6-{[4-(diéthylamino)-1-oxo-2-butén-1-yl]amino}-7-[(S)-(tétrahydrofuran-3-yl)oxy]-quinazoline,
(25) 4-[(3-chloro-4-fluorophényl)amino]-6-{[4-(4-(diéthylamino-méthyl-pipéridin-1-yl)-1-oxo-2-butén-1-yl]amino}-7-cyclo-propylméthoxy-quinazoline,
(26) 4-[(3-chloro-4-fluorophényl)amino]-6-{[4-(N-méthyl-N-cyclo-propylméthyl-amino)-1-oxo-2-butén-1-yl]amino}-7-cyclopropyl-méthoxy-quinazoline,
(27) 4-[(3-chloro-4-fluorophényl)amino]-6-({4-(N-méthyl-N-(2-méthoxypropyl)-amino]-1-oxo-2-butén-1-yl}amino)-7-cyclopropyl-méthoxy-quinazoline,
(28) 4-[(3-chloro-4-fluorophényl)amino]-6-({4-(N-méthyl-N-(3-méthoxypropyl)-amino]-1-oxo-2-butén-1-yl}amino)-7-cyclopropyl-méthoxy-quinazoline,
(29) 4-[(3-chloro-4-fluorophényl)amino]-6-{[4-(diéthylamino)-1-oxo-2-butén-1-yl]amino}-7-[(tétrahydropyran-4-yl)oxy]-quinazoline,
(30) 4-[(3-chloro-4-fluorophényl)amino]-6-({4-(N-méthyl-N-(1-méthoxy-2-propyl)-amino]-1-oxo-2-butén-1-yl}amino)-7-cyclopropyl-méthoxy-quinazoline,
(31) 4-[(3-chloro-4-fluorophényl)amino]-6-{[4-(N,N-diméthylamino)-1-oxo-2-butin-1-yl]amino}-7-cyclopropylméthoxy-quinazoline,
(32) 4-[(3-chloro-4-fluorophényl)amino]-6-{[4-(N,N-diéthylamino)-1-oxo-2-butin-1-yl]amino}-7-cyclopropylméthoxy-quinazoline,
(33) 4-[(3-chloro-4-fluorophényl)amino]-6-{[4-(pipéridin-1-yl)-1-oxo-2-butin-1-yl]amino}-7-cyclopropylméthoxy-quinazoline,
(34) 4-[(3-chloro-4-fluorophényl)amino]-6-{[4-(morpholin-4-yl)-1-oxo-2-butin-1-yl]amino}-7-cyclopropylméthoxy-quinazoline,
(35) 4-[(3-chloro-4-fluorophényl)amino]-6-{[4-(N,N-diméthylamino)-1-oxo-2-butén-1-yl]amino}-7-(2-cyclopropyl-éthoxy)-quinazoline,
(36) 4-[(3-chloro-4-fluorophényl)amino]-6-({4-[bis-(2-méthoxy-éthyl)-amino]-1-oxo-2-butén-1-yl}amino)-7-cyclobutyloxy-quinazoline,
(37) 4-[(3-chloro-4-fluorophényl)amino]-6-({4-[N-méthyl-N-(2-méthoxy-éthyl)-amino]-1-oxo-2-butén-1-yl}amino)-7-cyclobutyloxy-quinazoline,
(38) 4-[(3-chloro-4-fluorophényl)amino]-6-({4-[(S)-N-méthyl-N-(1-méthoxy-2-propyl)-amino]-1-oxo-2-butén-1-yl}amino)-7-cyclobutyloxy-quinazoline,
(39) 4-[(3-chloro-4-fluorophényl)amino]-6-({4-[(R)-N-méthyl-N-(1-méthoxy-2-propyl)-amino]-1-oxo-2-butén-1-yl}amino)-7-cyclobutyloxy-quinazoline,
(40) 4-[(3-chloro-4-fluorophényl)amino]-6-({4-[N-méthyl-N-(1-méthoxy-2-propyl)-amino]-1-oxo-2-butén-1-yl}amino)-7-cyclopropylméthoxy-quinazoline,
(41) 4-[(3-chloro-4-fluorophényl)amino]-6-({4-[N-méthyl-N-(2-méthoxypropyl)-amino]-1-oxo-2-butén-1-yl}amino)-7-cyclopropylméthoxy-quinazoline,
(42) 4-[(3-chloro-4-fluorophényl)amino]-6-({4-[N-méthyl-N-(3-méthoxypropyl)-amino]-1-oxo-2-butén-1-yl}amino)-7-cyclopropylméthoxy-quinazoline,
(43) 4-[(3-chloro-4-fluorophényl)amino]-6-{[4-(N-cyclopropyl-N-méthyl-amino)-1-oxo-2-butén-1-yl]amino}-7-cyclobutyloxy)-quinazoline,
(44) 4-[(3-chloro-4-fluorophényl)amino]-6-{[4-(N-cyclopropylméthyl-N-méthyl-amino)-1-oxo-2-butén-1-yl]amino}-7-cyclobutyloxy-quinazoline,
(45) 4-[(3-chloro-4-fluorophényl)amino]-6-{[4-(pyrrolidin-1-yl)-1-oxo-2-butén-1-yl]amino}-7-cyclobutyloxy-quinazoline,
(46) 4-[(3-chloro-4-fluorophényl)amino]-6-{[4-(2-méthyl-pyrrolidin-1-yl)-1-oxo-2-butén-1-yl]amino}-7-cyclobutyloxy-quinazoline,
(47) 4-[(3-chloro-4-fluorophényl)amino]-6-{[4-(2,5-diméthyl-pyrrolidin-1-yl)-1-oxo-2-butén-1-yl]amino}-7-cyclobutyloxy-quinazoline,
(48) 4-[(3-chloro-4-fluorophényl)amino]-6-{[4-(pipéridin-1-yl)-1-oxo-2-butén-1-yl]amino}-7-cyclobutyloxy-quinazoline,
(49) 4-[(3-chloro-4-fluorophényl)amino]-6-{[4-(2-méthyl-pipéridin-1-yl)-1-oxo-2-butén-1-yl]amino}-7-cyclobutyloxy-quinazoline,
(50) 4-[(3-chloro-4-fluorophényl)amino]-6-{[4-(2,6-diméthyl-pipéridin-1-yl)-1-oxo-2-butén-1-yl]amino}-7-cyclobutyloxy-quinazoline,
(51) 4-[(3-chloro-4-fluorophényl)amino]-6-{[4-(3-méthyl-morpholin-4-yl)-1-oxo-2-butén-1-yl]amino}-7-cyclobutyloxy-quinazoline,
(52) 4-[(3-chloro-4-fluorophényl)amino]-6-{[4-(3,5-diméthyl-morpholin-4-yl)-1-oxo-2-butén-1-yl]amino}-7-cyclobutyloxy-quinazoline,
(53) 4-[(3-chloro-4-fluorophényl)amino]-6-({4-[N-méthyl-N-(2-méthoxyéthyl)-amino]-1-oxo-2-butén-1-yl}amino)-7-(tétrahydrofuran-3-yl-oxy)-quinazoline,
(54) 4-[(3-chloro-4-fluorophényl)amino]-6-({4-[N-méthyl-N-(2-méthoxyéthyl)-amino]-1-oxo-2-butén-1-yl}amino)-7-(tétrahydropyran-4-yl-oxy)-quinazoline et
(55) 4-[(3-chloro-4-fluorophényl)amino]-6-({4-[N-méthyl-N-(2-méthoxyéthyl)-amino]-1-oxo-2-butén-1-yl}amino)-7-(tétrahydrofuran-2-yl-méthoxy)-quinazoline,
et leurs sels.

3. Sels physiologiquement acceptables des composés selon la revendication 1 ou 2, avec des acides ou des bases inorganiques ou organiques.

4. Médicament contenant un composé selon la revendication 1 ou 2, ou sel physiologiquement acceptable selon la revendication 3, parallèlement à éventuellement un ou plusieurs véhicules et/ou diluants inertes.

5. Utilisation d'un composé selon au moins l'une des revendications 1 à 3, pour la production d'un médicament qui est approprié pour le traitement de tumeurs bénignes ou malignes, pour la prévention et le traitement de maladies des voies respiratoires et des poumons et pour le traitement de maladies du tractus gastro-intestinal et des voies biliaires et de la vésicule biliaire.

6. Procédé de production d'un médicament selon la revendication 4, **caractérisé en ce que**, de façon non chimique, un composé selon au moins l'une des revendications 1 à 3 est incorporé dans un ou plusieurs véhicules et/ou diluants inertes.

7. Procédé de production des composés de formule générale 1 selon les revendications 1 à 3, **caractérisé en ce que**
a) l'on fait réagir un composé de formule générale 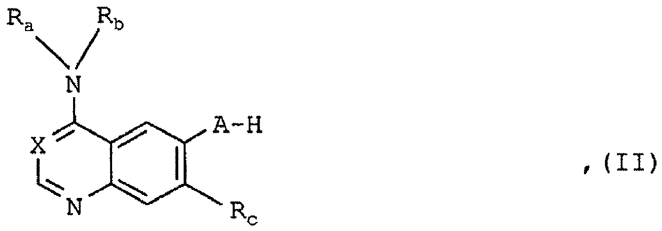 dans laquelle
Rₐ à R_{c}, A et X sont tels que définis dans les revendications 1 ou 2, avec un composé de formule générale
Z₁ - B - C - D - E , (III)
dans laquelle
B à E sont tels que définis dans les revendications 1 ou 2
et
Z₁ représente un groupe partant, ou
b) pour la production de composés de formule générale I dans laquelle le radical E est relié par un atome d'azote avec le radical D, on fait réagir un composé de formule générale 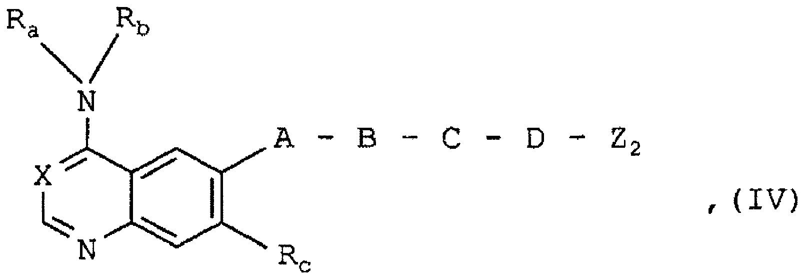 dans laquelle
Rₐ à R_{c}, A à D et X sont tels que définis dans les revendications 1 ou 2, et
Z₂ représente un groupe partant, avec un composé de formule générale
H - E' , (V)
dans laquelle
E' représente l'un des radicaux mentionnés pour E dans les revendications 1 ou 2, qui est relié par un atome d'azote au radical D, et
si on le souhaite, un composé de formule générale I ainsi obtenu, qui contient un groupe amino, alkylamino ou imino est converti par acylation ou sulfonylation en un composé acyle ou sulfonyle correspondant de formule générale I et/ou
un composé de formule générale I ainsi obtenu, qui contient un groupe amino, alkylamino ou imino est converti par alkylation ou alkylation réductrice en un composé alkyle correspondant de formule générale I et/ou
un composé de formule générale I ainsi obtenu, qui contient un groupe carboxy ou hydroxyphosphoryle, est converti par estérification en un ester correspondant de formule générale I et/ou
un composé de formule générale I ainsi obtenu, qui contient un groupe carboxy ou ester, est converti en le faisant réagir avec une amine correspondante en un amide correspondant de formule générale I et/ou
si nécessaire, un radical protecteur utilisé lors des réactions décrites précédemment est à nouveau clivé et/ou
si on le souhaite, un composé de formule générale I ainsi obtenu est séparé en ses stéréo-isomères et/ou
un composé de formule générale I ainsi obtenu est converti en ses sels, en particulier pour l'utilisation pharmaceutique en ses sels physiologiquement acceptables.
